(19) Europäisches Patentamt
European Patent Office
Office européen des brevets

(11) **EP 2 527 433 B1**

(12) **EUROPEAN PATENT SPECIFICATION**

(45) Date of publication and mention of the grant of the patent:
**26.08.2015 Bulletin 2015/35**

(51) Int Cl.:
***C12N 15/00*** *(2006.01)*  ***C12N 15/09*** *(2006.01)*
***C12N 15/87*** *(2006.01)*

(21) Application number: **11734560.3**

(22) Date of filing: **12.01.2011**

(86) International application number:
**PCT/JP2011/050307**

(87) International publication number:
**WO 2011/089953 (28.07.2011 Gazette 2011/30)**

(54) **FOREIGN GENE TRANSFER METHOD BY ELECTROPORATION TECHNIQUE**

FREMDGENÜBERTRAGUNGSVERFAHREN DURCH EINE ELEKTROPORATIONSTECHNIK

PROCÉDÉ D'INTRODUCTION DE GÈNE ÉTRANGER PAR PROCÉDÉ D'ÉLECTROPORATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.01.2010 JP 2010011881**

(43) Date of publication of application:
**28.11.2012 Bulletin 2012/48**

(73) Proprietor: **Nepa Gene Co., Ltd.**
**Ichikawa-shi, Chiba 272-0114 (JP)**

(72) Inventors:
• **HAYAKAWA Yasuhiko**
**Ichikawa-shi**
**Chiba 2720114 (JP)**
• **HAYAKAWA Kiyoshi**
**Ichikawa-shi**
**Chiba 2720114 (JP)**

(74) Representative: **Vossius & Partner**
**Patentanwälte Rechtsanwälte mbB**
**Siebertstrasse 3**
**81675 München (DE)**

(56) References cited:
**WO-A1-01/62337**

• **PAVSELJ ET AL: "DNA electrotransfer into the skin using a combination of one high- and one low-voltage pulse", JOURNAL OF CONTROLLED RELEASE, ELSEVIER, AMSTERDAM, NL, vol. 106, no. 3, 2 September 2005 (2005-09-02), pages 407-415, XP005038460, ISSN: 0168-3659**

• **SATKAUSKAS SAULIUS ET AL: "Electrophoretic component of electric pulses determines the efficacy of in vivo DNA electrotransfer.", HUMAN GENE THERAPY OCT 2005, vol. 16, no. 10, October 2005 (2005-10), pages 1194-1201, XP002709603, ISSN: 1043-0342**

• **BUREAU, M. F. ET AL.: 'Importance of association between permeabilization and electrophoretic forces for intramuscular DNA electrotransfer' BIOCHIMICA ET BIOPHYSICA ACTA vol. 1474, 2000, pages 353 - 359, XP004276575**

• **SATKAUSKAS, S. ET AL.: 'Mechanisms of in vivo DNA electrotransfer: respective contributions of cell electropermeabilization and DNA electrophoresis' MOLECULAR THERAPY vol. 5, no. 2, February 2002, pages 133 - 140, XP002378554**

• **KLENCHIN, V. A. ET AL.: 'Electrically induced DNA uptake by cells is a fast process involving DNA electrophoresis' BIOPHYS. J. vol. 60, October 1991, pages 804 - 811, XP008161568**

• **SUKHAREV, S. I. ET AL.: 'Electroporation and electrophoretic DNA transfer into cells' BIOPHYS. J. vol. 63, November 1992, pages 1320 - 1327, XP000646910**

• **KANDUSER, M. ET AL.: 'Mechanisms involved in gene electrotransfer using high- and low-voltage pulses -an in vitro study' BIOELECTROCHEMISTRY vol. 74, 2009, pages 265 - 271, XP025884686**

EP 2 527 433 B1

- YUSUKE YAMAMOTO ET AL.: 'Denki Pulse-ho o Mochiita Idenshi Donyu Koritsu no Saitekika' 2010 NENDO JAPAN SOCIETY FOR BIOSCIENCE, BIOTECHNOLOGY, AND AGROCHEMISTRY TAIKAI KOEN YOSHISHU vol. 3AXA11, March 2010, page 235, XP008169196

- CEPURNIENE, K. ET AL.: 'Influence of plasmid concentration on DNA electrotransfer in vitro using high-voltage and low-voltage pulses' J. MEMBRANE BIOL vol. 236, no. 1, July 2010, pages 81 - 85, XP019840553

**Description**

Technical Field

This invention relates to a method for transferring an extraneous gene by an electroporation technique, and more particularly, to a method for transferring an extraneous gene by an electroporation technique, which is remarkably improved in viability and gene transferring rate, the method including continuously applying, to an animal cell, a first electric pulse (strong electric pulse) and a second electric pulse (weak electric pulse) under specific conditions.

Background Art

The gene transferring method is classified into two methods, the virus vector method and the non-virus vector method. As the non-virus vector method for transferring an extraneous gene into animal cells of fertilized egg, blood corpuscle, skin, muscle, internal organs, etc., there are various methods such as the microinjection method, the particle gun method, the hydrodynamic - method, the sonoporation method and the electroporation method. And as the method for transferring an extraneous gene into suspension (culture) cells (cells suspended in the solution), there are the lipofection method, the sonoporation method and the electroporation method. In addition, as the method for transferring an extraneous gene into adherent cells (cells adhered to petri dish, well plate, etc.), there are the lipofection method, the phosphoric acid method, the DAEA dextran method, and the microinjection method.

Among the above methods, the electroporation method is a method to make temporally a micro hole in the cell membrane by applying a high voltage electric pulse so that the extraneous DNA such as plasmid can pass through the hole and be taken in the cells. This method is highly evaluated as the most advantageous and productive gene transferring method among the others from various view points. This method has various advantageous features such as wide applicability to various living things including plants, high gene transferring rate, excellent reproducibility, easier operation, no need to use special reagents, and possibility to treat many cells at the same time.

The electroporation technique is more effective gene transferring method comparing to the method such as the phosphoric acid method, although the gene transferring rate of the electroporation technique is still lower and not sufficient. Depending on the kind of cells, the electroporation technique achieves only extremely lower transferring rate.

In the conventional electroporation technique, one time of the electric pulse delivered from the exponential output device, or one or more times of electric pulses (at fixed constant higher voltage) delivered from the square pulse type electric pulse outputting device is applied for gene transferring.

In the case of applying one time of the electric pulse delivered from the exponential output device, it is inevitably needed to apply so strong electric pulse that might kill at least 50% of the cells. And its gene transferring rate remains very low, only 1-10% of the survived cells, even only 30% in the best case. Further, in the case of the square pulse type electric pulse outputting device, it is needed to apply the strong electric pulse that might kill at least 20% of the cells. And its gene transferring rate remains very low, only 1-15% of the survived cells, even only 30% in the best case (see Non-Patent Literature 1).

And it is possible to increase the gene transferring rate by applying stronger electric pulse, but it affects the viability of the cells and decreases extremely the number of the gene transferred cells actually obtained.

In the electroporation using the conventional electric pulse outputting device, use of the specialized buffer for electroporation is needed essentially, resulting in high running cost. And without specialized buffer, these methods were not applicable because of extremely lower efficiency.

Citation List

Non Patent Literature

Non-Patent Literature 1: Biotechniques Vol.17, No.6 (1994) "Short Technical Report"

The prior art includes KANDUSER, M. ET AL.: "Mechanisms involved in gene electrotransfer using high- and low-voltage pulses -an in vitro study", BIOELECTROCHEMISTRY, vol. 74, 2009, pages 265-271, which discloses a dual pulse in vitro electroporation means for delivery of nucleic acid to cells. In this paper, a first high voltage pulse of 800V/cm, given for 4 x 100µs was used, with the distance between the electrodes delivering the pulse being 2mm. This was followed by low voltage pulsing.

**EP 2 527 433 B1**

Summary of Invention

Technical Problem

An object of this invention is to provide a method for transferring an extraneous gene by an electroporation technique, which solves the above problems, is applicable to a wide range of animal cells, and is extremely remarkably improved in viability and gene transferring rate.

Another object of this invention is to provide a method for transferring an extraneous gene by an electroporation technique with high viability and gene transferring rate even in the case where no specialized electroporation buffer is used.

Solution to Problem

The inventors of this invention have made extensive studies. As a result, the inventors have found that significantly improved viability and gene transferring rate can be achieved by a method for transferring an extraneous gene into an animal cell by an electroporation technique, the method including continuously applying a first electric pulse (strong electric pulse) and a second electric pulse (weak electric pulse) under specific conditions.

The inventors also have found that the method allows extraneous gene to be transferred with high viability and gene transferring rate even in the case where a liquid medium capable of being used for culturing of the animal cell is used as an electroporation buffer (in the case where no specialized buffer is used).

Note that a possible principle for the method is as follows: a first electric pulse (stronger electric pulse) is first applied to make a micro hole in the cell membrane of a targeted animal cell, thereby transferring a nucleic acid into the animal cell, and a second electric pulse (weaker electric pulse) is then applied, thereby further transferring a nucleic acid into the animal cell and simultaneously restoring the cell membrane positively.

This invention has been completed based on those findings.

The invention is the following :

1. A method for transferring an extraneous gene into an animal cell by an electroporation technique, the method comprising:

(a) preparing a solution containing suspended animal cells and the extraneous gene to be transferred into the animal cell;
(b) filling a 1mm gap cuvette electrode, a 2mm gap cuvette electrode, or a 4mm gap cuvette electrode with the solution;
(c) applying, to the animal cell suspended in the solution, a first electric pulse having an electric field strength of 500 to 875 V/cm so that a total calorie strength is 1.39 to 4.37 J/100 $\mu$L; and
(d) applying a second electric pulse having an electric field strength of 50 to 250 V/cm so that a calorie strength per pulse is 0.13 to 3.57 J/100 $\mu$L,

wherein methods of treatment of the human or animal body by surgery or therapy are excluded.

2. A method for transferring an extraneous gene according to item 1, wherein the applying of the second electric pulse is carried out two or more times.

3. A method for transferring an extraneous gene according to item 1 or 2, wherein the applying of the second electric pulse is carried out less than one minute after the applying of the first electric pulse.

4. A method for transferring an extraneous gene according to any one of items 1 to 3, wherein the animal cell is a mammalian cell.

The disclosure made relates to a method for transferring an extraneous gene into an animal cell by an electroporation technique, the method including: applying, to the animal cell, a first electric pulse having an electric field strength of at least 300 V/cm or more so that a total calorie strength is 0.2-40 J/100 $\mu$L; and applying a second electric pulse having an electric field strength of at least 15 V/cm or more so that a calorie strength per pulse is 0.01-5 J/100 $\mu$L.

The disclosure made also relates to a method for transferring an extraneous gene according to the first aspect, in which the applying of the second electric pulse is carried out twice or more.

The disclosure made also relates to a method for transferring an extraneous gene according to the first or the second aspect, in which the applying of the second electric pulse is carried out less than one minute after the applying of the first electric pulse.

4

The disclosure made also relates to a method for transferring an extraneous gene according to any one of the first to the third aspect, in which the animal cell includes a mammalian cell.

The disclosure made also relates to amethod for transferring an extraneous gene according to any one of the first to the fourth aspects, in which the animal cell includes an animal cell suspended in a solution.

The disclosure made also relates to a method for transferring an extraneous gene according to any one of the first to the fifth aspects, in which the solution includes a liquid medium capable of being used for culturing of the animal cell.

Advantageous Effects of Invention

This invention provides the method for transferring an extraneous gene by an electroporation technique, which is applicable to a wide range of animal cells (in particular, vertebrate and insect cells) and is extremely remarkably improved in viability and gene transferring rate.

Thus, this invention allows extraneous gene to be transferred with high viability and gene transferring rate even in the case where a liquid medium capable of being used for culturing of the above cells is used as an electroporation buffer (in the case where no expensive specialized buffer is used). That is, this invention allows running costs to be reduced significantly.

This invention also allows extraneous gene to be efficiently transferred into primary cells, ES cells, some cell lines and non-adherent cells (e.g., lymphoid lineage cells and some cancer cells), in each of which it has been difficult to achieve gene transferring by the conventional electroporation technique.

This invention also allows animal gene transferred cells (e. g. , iPS cells) useful in a wide range of industrial fields to be prepared efficiently at low cost.

Brief Description of Drawings

[Fig. 1] Photo images showing the detected fluorescent-labeled protein originated from the transferred DNA in Example 28.

[Fig. 2] Photo images showing the detected fluorescent-labeled protein originated from the transferred DNA in Example 28.

[Fig. 3] Photo images showing the detected fluorescent-labeled protein originated from the transferred DNA in Example 28.

[Fig. 4] Photo images showing the detected fluorescent-labeled protein originated from the transferred DNA in Example 28.

[Fig.5] Photo images showing the detected fluorescent-labeled protein originated from the transferred DNA in Example 28.

[Fig. 6] Photo images showing the detected fluorescent-labeled protein originated from the transferred DNA in Example 28.

[Fig.7] Photo images showing the detected fluorescent-labeled protein originated from the transferred DNA in Example 28.

[Fig. 8] Photo images showing the detected fluorescent-labeled protein originated from the transferred DNA in Example 28.

[Fig. 9] Photo images showing the detected fluorescent-labeled protein originated from the transferred DNA in Example 28.

[Fig. 10] Photo images showing the detected fluorescent-labeled protein originated from the transferred DNA in Example 28.

[Fig. 11] Photo images showing the detected fluorescent-labeled protein originated from the transferred DNA in Example 28.

[Fig. 12] Photo images showing the detected fluorescent-labeled protein originated from the transferred DNA in Example 28.

[fig. 13] Photo images showing the detected fluorescent-labeled protein originated from the transferred DNA in Example 28.

[Fig. 14] Photo images showing the detected fluorescent-labeled protein originated from the transferred DNA in Example 28.

[Fig. 15] Photo images showing the detected fluorescent-labeled protein originated from the transferred DNA in Example 28.

[Fig. 16] Photo images showing the detected fluorescent-labeled protein originated from the transferred DNA in Example 28.

[Fig. 17] Photo images showing the detected fluorescent-labeled protein originated from the transferred DNA in Example 28.

[Fig. 18] Photo images showing the detected fluorescent-labeled protein originated from the transferred DNA in Example 28.

[Fig. 19] Photo images showing the detected fluorescent-labeled protein originated from the transferred DNA in Example 28.

[Fig. 20] Photo images showing the detected fluorescent-labeled protein originated from the transferred DNA in Example 28.

[Fig. 21] Photo images showing the detected fluorescent-labeled protein originated from the transferred DNA in Example 28.

[Fig. 22] Photo images showing the detected fluorescent-labeled protein originated from the transferred DNA in Example 28.

[Fig. 23] Photo images showing the detected fluorescent-labeled protein originated from the transferred DNA in Example 28.

[Fig. 24] Photo images showing the detected fluorescent-labeled protein originated from the transferred DNA in Example 28.

[Fig. 25] Photo images showing the detected fluorescent-labeled protein originated from the transferred DNA in Example 28.

[Fig. 26] A photo image showing the detected fluorescent-labeled protein originated from the transferred DNA in Example 28.

[Fig. 27] Photo images showing the detected fluorescent-labeled protein originated from the transferred DNA in Example 28.

[Fig. 28] Photo images showing the detected fluorescent-labeled protein originated from the transferred DNA in Example 28.

[Fig. 29] A photo image showing the detected fluorescent-labeled protein originated from the transferred DNA in Example 28.

[Fig. 30] Photo images showing the detected fluorescent-labeled protein originated from the transferred DNA in Example 28.

[Fig. 31] Photo images showing the detected fluorescent-labeled protein originated from the transferred DNA in Example 28.

[Fig. 32] Photo images showing the detected fluorescent-labeled protein originated from the transferred DNA in Example 28.

[Fig. 33] Photo images showing the detected fluorescent-labeled protein originated from the transferred DNA in Example 28.

[Fig. 34] Photo images showing the detected fluorescent-labeled protein originated from the transferred DNA in Example 28.

[Fig. 35] Photo images showing the detected fluorescent-labeled protein originated from the transferred DNA in Example 28.

[Fig. 36] A photo image showing the detected fluorescent-labeled protein originated from the transferred DNA in Example 29.

Description of Embodiments

Hereinafter, this invention is described in detail.

This invention relates to a method for transferring an extraneous gene by an electroporation technique, and more particularly, to a method for transferring an extraneous gene by electroporation, which is remarkably improved in viability and gene transferring rate, the method including continuously applying, to an animal cell, a first electric pulse (strong electric pulse) and a second electric pulse (weak electric pulse) under specific conditions.

<Device and method for outputting electric pulse>

In this invention, any conventional square pulse type electric pulse outputting device (electroporator) can be used by devising its usage as long as the device can output a first electric pulse and a second electric pulse under specific conditions (two stepped electric pulses under specific conditions) to be described later.

For example, there are given square pulse type outputting devices such as Gene Pulser Xcell (BioRad) and ECM830 (BTX). These devices can output continuously electric pulses set so as to have the same voltage and pulse length, but cannot output continuously electric pulses set so as to have different voltages and pulse lengths (two stepped electric pulses under specific conditions). Thus, two stepped electric pulses under specific conditions are output by a method including outputting a first electric pulse from the first device of two devices arranged side by side, changing the connection of a cuvette electrode holder to the second device, and a few seconds later, outputting a second electric pulse. Alternatively, in the case where a single device is used, there may be employed a method including outputting a first electric pulse, setting new conditions for a second electric pulse, and a few seconds later, outputting the second electric pulse.

Note that in this invention, the conventional square pulse type electric pulse outputting device (e.g., an outputting device such as Gene Pulser Xcell (BioRad) and ECM830 (BTX)) may be used by devising its usage as described above, but preferably, it is desired to use a specialized device which can output a first electric pulse and a second electric pulse under specific conditions (two stepped electric pulses under specific conditions) to be described later.

An operation of applying an electric pulse to cells in this invention is carried out through the use of a cuvette electrode holder connected to an electric pulse outputting device and an electrode container for holding a cell/nucleic acid mixed solution (cuvette electrode).

An electric pulse output from the electric pulse outputting device is output through the container for holding the cuvette electrode to the cuvette electrode inserted in the electrode holder, and is delivered into the cells in the electrode container.

As the cuvette electrode, any cuvette electrode may be used as long as it has a capacity for general applications. For example, there are given 1 mm gap (capacity: 20-70 $\mu$m), 2 mm gap (capacity: 40-400 $\mu$m) and 4 mm gap (capacity: 80-800 $\mu$m).

In this invention, the operation is performed by filling the container with a solution containing targeted animal cells and extraneous gene (nucleic acid) to be transferred.

Herein, as the 'solution,' there may be used a conventional buffer and a liquid medium in which targeted animal cells can be proliferated (e.g., an MEM medium, a DMEM medium, an Opti-MEM medium, an $\alpha$-MEM medium, an RPMI-1640 medium, a DMEM/F-12 medium, a Williams medium or an ES medium) as well as a buffer capable of being used for the conventional electroporation technique, such as PBS or HEPES. Note that a less serum concentration is preferred in any of these liquid media in terms of increasing the gene transferring rate, and in particular, it is desired to use a 'serum-free medium.' Further, it is preferred to use a medium containing no antibiotic.

Note that the serum and the antibiotic can be added freely to the medium after the application of the electric pulse.

Herein, the 'extraneous gene' refers to a wide range of extraneous nucleic acid sequences intended to be transferred, and for example, refers to not only a full-length sequence (cDNA sequence and genome sequence) but also a partial sequence, a regulatory region, a spacer region, a mutated sequence and a construct of a gene. In particular, gene transfer using a vector DNA, an oligonucleotide (antisense, siRNA) or a virus vector is widely applied.

The amount of the nucleic acid (specifically, DNA) contained in the solution may be such an amount that the conventional electroporation technique is applicable. However, the amount is suitably 0.01-1 $\mu$g/$\mu$L, particularly suitably about 0.03-0.2 $\mu$g/$\mu$L from the viewpoint of increasing the viability and gene transferring rate.

A case where the amount of the nucleic acid is too large is not preferred because the viability lowers. On the other hand, a case where the amount of the nucleic acid is too small is not preferred because the gene transferring rate lowers.

In the case where the targeted animal cells are adherent cells, it is desired to treat the cells in an adherent state with trypsin or the like for separating the adhered cells to make a suspension, remove the trypsin and then mix the cells into a serum-free medium for electroporation.

Further, in the case where the animal cells are normally in a suspended state like blood cells, it is desired to wash the animal cells with an appropriate solution (e.g., a PBS buffer) and then mix the animal cells into a serum-free medium for electroporation.

From the viewpoint of improving the gene transferring rate, it is desired to subject the solution containing the animal cells and the nucleic acid to an operation such as pipetting or stirring with a vortex mixer for 1-2 seconds, to thereby sufficiently mix the animal cells and the nucleic acid in the solution. The number of the cells to be suspended is about $10^4$-$10^8$ cells/100 $\mu$L, preferably about $10^5$-$10^7$ cells/100 $\mu$L. Note that it is not preferred to foam the solution by excessively performing the operation such as stirring.

The operation of applying the electric pulse can be performed at room temperature (e.g., about 15-40°C). Note that it is preferred to avoid cooling with ice for preventing a water droplet from adhering to a metal (aluminum) part of the electrode container.

<Condition of electric pulse>

In this invention, both the viability and the gene transferring rate can be drastically improved as compared to the conventional electroporation technique by continuously applying, to a targeted animal cell, a first electric pulse and a second electric pulse under specific conditions (two stepped electric pulses under specific conditions).

The first electric pulse and the second electric pulse in this invention refer to such electric pulses that both the 'electric field strength' and the 'calorie strength' fall within a specific range to be described later.

On the other hand, when any one of the electric field strength and the calorie strength does not fall within the specific range, no sufficient effect can be obtained.

Herein, the "electric field strength" is a value indicating a voltage V to be applied per unit cm of an electrode gap (e.g., a cuvette electrode gap) in the electrode container as indicated by Equation 1. Its unit is indicated as (V/cm).

For example, in order to provide an electric field strength of 300 V/cm, a voltage of 30 V has only to be applied in a 1 mm gap cuvette (electrode gap: 1 mm), a voltage of 60 V has only to be applied in a 2 mm gap cuvette (electrode gap: 2 mm), and a voltage of 120 V has only to be applied in a 4 mm gap cuvette (electrode gap: 4 mm).

[Math. 1]

$$\text{Electric field strength (V/cm)} = \text{Voltage (V)} / \text{Electrode gap (cm)} \cdots$$

$$(\text{Equation 1})$$

Further, the "calorie strength" is a value indicating a calorie J to be applied per 100 μL of the solution (electroporation buffer) as indicated by Equation 2. Its unit is indicated as (J/100 μL). Note that the calorie (J) is a value indicated by the product of a voltage, a current and a time as indicated by Equation 3.

For example, when a voltage of 150 V with a pulse length of 5 m sec is applied to 100 μL of a solution (electroporation buffer) having an electric impedance of 50 Q, a current of 3 A is generated. As a result, the calorie to be applied per 100 μL of the solution is 2.25 (J/100 μL).

Note that even in the case where the voltage, the capacity (electric impedance), the pulse length (time) and the like are changed, similar results (viability and gene transferring rate) can be obtained as long as the electric field strength and the calorie strength are kept constant.

[Math. 2]

$$\text{Calorie strength (J/100 μL)} = \text{Calorie (J)} / \text{Solution volume (100 μL)}$$

$$\cdots (\text{Equation 2})$$

[Math. 3]

$$\text{Calorie (J)} = \text{Voltage (V)} \times \text{Current (A)} \times \text{Time (sec)} \cdots (\text{Equation 3})$$

The "first electric pulse" in this invention is a strong electric pulse to be applied in order to make a micro hole in the cell membrane of an animal cell to transfer an extraneous nucleic acid (DNA, RNA) into the cell through the micro hole. The "first electric pulse" allows a large amount of DNA to be transferred into the cytoplasm through the cell membrane, but causes major damage in the cell membrane

In sofar as the disclosure made is concerned, the electric field strength of the first electric pulse can be at least 300 V/cm or more, preferably 375 V/cm or more. Note that when the electric field strength is less than this level, no sufficient gene transferring rate can be obtained. In sofar as the disclosure made is concerned, the upper limit of the electric field strength has only to be such a value that the viability of cells does not remarkably lower, and it is desired that the upper limit be, for example, 15, 000 V/cm or less, preferably 7, 500 V/cm or less, more preferably 5,000 V/cm or less, most preferably 4,500 V/cm or less.

In sofar as the disclosure made is concerned, it is desired that the 'total calorie strength' of the first electric pulse be 0.2 J/100 μL or more, preferably 0.25 J/100 μL or more, more preferably 0.3 J/100 μL or more. Further, in sofar as the disclosure made is concerned, it is desired that the upper limit of the total calorie strength to be applied be 40 J/100 μL or less, preferably 20 J/100 μL or less, particularly preferably 17 J/100 μL or less, more particularly preferably 7 J/100 μL or less (Note that the upper limit is 5.3 J/100 μL or less for Hela cells, in particular).

The frequency of the first electric pulse to be applied may be any frequency as long as the total calorie strength of the electric pulse falls within the required range. For example, an electric pulse having a calorie strength within the required range may be applied one time. Alternatively, an electric pulse having a calorie strength less than that required may be applied ten times so that the total calorie strength falls within the range of the calorie strength required.

The second electric pulse in this invention is applied after the application of the first electric pulse (output of the last pulse of the first electric pulse). An interval between the pulses may be an extremely long interval such as about 10 minutes. However, the interval is preferably less than one minute from the viewpoint of improving the viability. The interval is particularly preferably less than 100 milliseconds.

The "second electric pulse" in this invention is a weak electric pulse to be applied in order to transfer the remaining DNA, which has not been transferred by the application of the first electric pulse through the micro hole of the cell membrane, into the cytoplasm through the cell membrane, and to restore the cell membrane positively to elevate the viability of cells (to provide a healing effect).

In sofar as the disclosure made is concerned, the 'electric field strength' of the second electric pulse can be at least 15 V/cm or more, preferably 25 V/cm or more. Note that the upper limit of the electric field strength has only to be such a value that the viability of cells does not remarkably lower, and it is desired that the upper limit be, for example, 300 V/cm or less, preferably 150 V/cm or less.

In sofar as the disclosure made is concerned, it is desired that the 'calorie strength' of the second electric pulse be 0.01 J/100 μL or more, preferably 0.02 J/100 μL ormore, more preferably 0. 09 J/100 μL or more, and it is desired that the upper limit of the calorie strength be 5 J/100 μL or less, preferably 4.5 J/100 μL or less, more preferably 3.6 J/100 μL or less.

Further, as the frequency of the second electric pulse, the electric pulse within the above range may be applied one time. However, a significant improvement in viability is remarkably found when the application is performed preferably two or more times, particularly preferably three or more times, more particularly preferably five or more times, most preferably ten or more times. Note that even when the application is performed more than ten times, no particularly significant change in viability is found.

Gene transferred cells with high viability and gene transferring rate can be obtained by culturing, in a general medium, the cells obtained after the application of the electric pulses within the specific range described above.

Note that in the case where a serum-free medium is used as an electroporation buffer, the cells can be directly collected in the medium containing serum and an antibiotic after the application of the electric pulses. In other words, the cells can be collected without causing any damage and loss of the cells due to liquid exchange and then cultured.

<The targeted cells>

The electroporation in this invention can be applied to a wide range of animal cells.

Here the term "animal cells" means the cells of eukaryotic multiple cells creature classified as the 'animal' in taxonomy. Examples are Deuterostomes such as vertebrates (mammals, birds, reptiles, amphibias, fishes, etc.), chordates (ascidians, etc.), hemichordates (balanoglossus, etc.), and echinoderms (starfishes, sea cucumbers, etc.); Protostomia such as arthropods (insects, crustaceans, etc.), mollusks (shellfishes, squids, etc.), nematodes (roundworms, etc.), annelidas (earthworms, etc.), and flatworms (planarians, etc.); Diploblastic animals such as cnidarians (jellyfishes, coral, etc.); and No blastoderm animals such as porifera (sponges, etc.).

The electroporation in this invention is most effectively applied especially to any animals classified in Vertebrates such as mammals, birds, reptiles, amphibias, fishes, etc. , and Arthropods such as insects. And its higher effectiveness to the animals is expected to have wide and versatile industrial applications, because its higher effectiveness was confirmed with mammals (human, horse, mouse, rat and hamster), insects (drosophila) and amphibias (soft shelled turtle) as written in Examples described later.

The electroporation in this invention basically can be applied to the "animal cells" of any organs and tissues of animals. For instances, it can be effectively applied to cell lines of stem cells, cancer cells, or normal cells, as well as primary cells as written below.

For examples, it can be applied to various 'stem cells' such as embryonic stem cells (ES cells), neural stem cells, hematopoietic stem cells, mesenchymal stem cells, hepatic stem cells, pancreatic stem cells, skin stem cells, muscle stem cells, germ stem cells, etc.

And it can be applied to 'cancer cells' including various cancer cells and oncocytes such as breast cancer cells, cervical cancer cells, pancreas cancer cells, liver cancer cells, lung cancer cells, epithelial cancer cells, esophagus cancer cells, prostate cancer cells, leukemia cells, sarcoma cells, lymphoma cells, etc.

And also, it can be applied to 'normal cells" such as primary cells or various tissue cells differentiated normally. For examples, it can be applied to hemocytes, lymphocytes (T lymphocytes, B lymphocytes, macrophages, etc.), epidermic cells (epidermic cells, keratinocytes, endothelial cells, etc.), connective tissue cells (fibroblasts, etc.), muscular tissues (myoblasts, skeletal muscle cells, tunica muscular cells, myocardial cells, etc.), neural cells (neuron, neuroblastoma, glial cells, etc.), various organ cells (kidney cells, lung cells, pancreatic cells, etc.), osseous tissues (osteoblasts, bone cells, cartilage cells, etc.), and germ cells (oocytes, spermatocyte, etc.).

In addition, the electroporation in this invention can be effectively applied to the primary cells, in which it has been difficult to achieve gene transferring, such as human amniotic mesenchymal cells, mouse neurons (embryonic day 14 cerebral cortex), mouse neurons (embryonic day 14 hippocampus), mouse neural stem cells, mouse embryonic fibroblasts, rat medullary neurons, rat meningeal fibroblasts, and rat olfactory ensheathing cells; and to the ES cells, in which it has been difficult to achieve gene transferring, such as mouse TT2 ES cells and ddy mouse ES cells; and to the cell lines, in which it has been difficult to achieve gene transferring, such as human embryonic lung fibroblasts (TIG-7), human immortalized fibroblasts (SUSM-1), human fibrosarcoma cells (HT1080), human pancreatic carcinoma cells (MIA-PaCa-2), human hepatoma cells (HepG2), human squamous carcinoma cells (HSC-2), human breast cancer cells (MCF-7), human esophageal carcinoma cells (TE-1), human prostate carcinoma cells (LNCaP), human ovarian carcinoma cells (OVCAR-3), human neuroblastoma cells (SK-N-SH), human B-cells precursor leukemia cells (Nalm-6), human burkitt's lymphoma cells (Raji), mouse embryonic fibroblasts (MEF), mouse macrophage cells (RAW264.7), mouse pancreatic $\beta$ cells (MIN6), rat embryonic fibroblasts (REF), and rat ventricular myoblasts (H9c2).

This invention is most effectively applied especially to primary cells, cancer cells, neural cells, and stem cells in the cells listed above.

Examples

We will explain our invention in detail by introducing many examples hereunder. But applicable field of our invention is never limited in the applications introduced as examples below.

<Example 1> Effects of the first electric pulse and the second electric pulse

(1) Preparation of cells

A medium was removed from a culture vessel in which HeLa Cells (human cervical cancer cell line: adherent cells) were cultured. The cells were then washed two or more times with a 0.02% EDTA-PBS solution for eliminating the influence of serum contained in the medium. The cells in an adherent state were then separated by trypsin treatment.

After confirming the separation of the cells, trypsin was removed by adding the same volume of an electroporation buffer (ES medium as a serum/antibiotic-free medium (NISSUI PHARMACEUTICAL CO., LTD.)) as that of the enzyme liquid used for the trypsin treatment and centrifuging the mixture (~1,000 rpm, 5 min).

The supernatant was then discarded. The separated cells were dispersed in the electroporation buffer, and 50 $\mu$L of the dispersion was sampled to measure the number of the cells with a hemocytometer. Centrifugation (~1, 000 rpm, 5 min) was performed again for removing the residual supernatant, and the electroporation buffer was added again to the collected cells to prepare a suspension at $1\times10^7$ cells/900 $\mu$L.

Next, 100 $\mu$L of a DNA solution (pCMV-EGFP vector, concentration: 1 $\mu$g/$\mu$L) was added to the above suspension so that the total volume was 1,000 $\mu$L. 100 $\mu$L each of the resultant suspension (cell: $1\times10^6$ cells, DNA: 0.1 $\mu$g/$\mu$L) was put into a 2 mm gap cuvette after careful sufficient stirring not to cause bubbling.

Note that in the case where the number of the cells was small, the electroporation buffer was added to the cells to prepare a suspension at $2.5\times10^6$ cells/450$\mu$L during the adjustment of the number of the cells, and 50 $\mu$L of the DNA solution (pCMV-EGFP vector, concentration: 1 $\mu$g/$\mu$L) was added so that the total volume was 500 $\mu$L, and 50 $\mu$L each of the resultant suspension (cell: 2. $5\times10^5$ cells, DNA: 0.1 $\mu$g/$\mu$L) was put into a 2 mm gap cuvette.

(2) Electric pulse treatment

The cuvette into which the cells prepared as above were put was inserted in a cuvette electrode chamber of an NEPA21 electroporator (NEPA GENE Co., Ltd.) and an electric pulse was output from the NEPA21 electroporator.

As shown in Tables 1, electroporation was then performed under the conditions of different combinations of the presence or absence of a first electric pulse and a second electric pulse and different frequencies of the second electric pulse (samples 53-56, 60). Note that this treatment was done at room temperature without cooling with ice for preventing a water droplet from adhering to the cuvette.

Note that a sample, which was not subjected to electric pulse treatment after put into the cuvette, was used as a control (sample 52).

After the electric pulse treatment, an MEM medium containing serum and an antibiotic was put into the cuvette. The whole volume of the liquid (including the cells after the electric pulse treatment) was then collected with a syringe, added to a culture plate filled with the MEM medium containing serum and an antibiotic and cultured under usual conditions (37°C, carbon dioxide concentration: 5%). The viability (calculated with Equation 4) was calculated. Further, the gene transferring rate (calculated with Equation 5) was calculated by detecting a fluorescent protein EGFP. The result is shown in Tables 1.

Note that in the following results, for Hela cells, such a condition that both of the viability and the gene transferring rate are 40% or more can be determined to be suitable. Further, for a series of cells, in each of which it is extremely difficult to achieve gene transferring, such a condition that both the values are about 10% can also be determined to be suitable.

[Math. 4]

$$\text{Viability} = \text{Number of viable cells after electric pulse treatment} / \text{Number of cells before electric pulse treatment} \times 100 \quad \cdots \quad (\text{Equation 4})$$

[Math. 5]

$$\text{Gene transferring rate} = \text{Number of gene transferred cells} / \text{Number of viable cells after electric pulse treatment} \times 100 \quad \cdots \quad (\text{Equation 5})$$

The results show that both of the viability and the gene transferring rate are drastically improved by continuously applying the suitable first electric pulse and second electric pulse.

Note that in the case where the first electric pulse was not applied, gene transferring itself did not occur. This result suggests that a process for applying the first electric pulse is a process essential for making a micro hole in the cell membrane to transfer extraneous DNA through the hole into cells.

The results also show that the viability is significantly improved by applying the second electric pulse, and that the viability is additionally improved by increasing the frequency of the second electric pulse (two or more times, optimally 10 times). This result suggests that the second electric pulse has an effect of accelerating the restoration of the micro hole formed by applying the first electric pulse.

[Table 1-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100 $\mu$l) per one pulse | |
| 52 | 2mm | 0.10 | 100 | - | - | - | - | - | - | - |
| 53 | 2mm | 0.10 | 100 | 36 | 0 | 0 | 0 | 0.000 | 0.000 | - |
| 54 | 2mm | 0.10 | 100 | 35 | 125 | 625 | 5 | 2.232 | 2.232 | - |
| 55 | 2mm | 0.10 | 100 | 34 | 125 | 625 | 5 | 2.298 | 2.298 | 50 |
| 56 | 2mm | 0.10 | 100 | 36 | 125 | 625 | 5 | 2.170 | 2.170 | 50 |
| 60 | 2mm | 0.10 | 100 | 34 | 125 | 625 | 5 | 2.298 | 2.298 | 50 |

[Table 1-B]

| Sample | Second electric pulse | | | | | | | Viability (%) | Gene transferring rate (%) |
| --- | --- | --- | --- | --- | --- | --- | --- | --- | --- |
| | Voltage (V) | Electric field strength (V/cm) | Pulse length (ms) | Pulse internal (ms) | Number of pulses | Calorie strength (J/100 $\mu$l) total | Calorie strenght (J/100 $\mu$l) per one pulse | | |
| 52 | - | - | - | - | - | - | - | >90 | 0 |
| 53 | 20 | 100 | 50 | 50 | 10 | 5.556 | 0.556 | 90 | 0 |
| 54 | 0 | 0 | 0 | - | 0 | 0.000 | 0.000 | 50 | 79 |
| 55 | 20 | 100 | 50 | - | 1 | 0.588 | 0.588 | 80 | 86 |
| 56 | 20 | 100 | 50 | 50 | 2 | 1.111 | 0.556 | 90 | 86 |
| 60 | 20 | 100 | 50 | 50 | 10 | 5.882 | 0.588 | >90 | 86 |

<Example 2> Examination of voltage of the first electric pulse (1)

As shown in Tables 2, the first electric pulse was applied by varying its voltage. Other conditions for electroporation were same to Example 1 (samples 2-11).

Note that a sample, which was not subjected to electric pulse treatment after put into the cuvette, was used as a control (sample 1).

And the viability and the gene transferring rate were calculated in the same manner as in Example 1. The result is shown in Tables 2.

The result showed that both the viability and the gene transferring rate were as high as 50% or more, when voltage of the first electric pulse was adjusted so as to be controlled in the range of electric field strength=500-875 V/cm and total calorie strength=1.39-4.37 J/100 $\mu$L. Especially in the case of electric field strength=500-750 V/cm and total calorie strength=1.39-3.21 J/100 $\mu$L, the viability and the gene transferring rate were as high as 80% or more.

[Table 2-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100$\mu$l) per one pulse | |
| 1 | 2mm | 0.10 | 100 | - | - | - | - | - | - | - |
| 2 | 2mm | 0.10 | 100 | 35 | 25 | 125 | 5 | 0.089 | 0.089 | 50 |
| 3 | 2mm | 0.10 | 100 | 35 | 50 | 250 | 5 | 0.357 | 0.357 | 50 |
| 4 | 2mm | 0.10 | 100 | 34 | 75 | 375 | 5 | 0.827 | 0.827 | 50 |
| 5 | 2mm | 0.10 | 100 | 36 | 100 | 500 | 5 | 1.389 | 1.389 | 50 |
| 6 | 2mm | 0.10 | 100 | 40 | 125 | 625 | 5 | 1.953 | 1.953 | 50 |
| 7 | 2mm | 0.10 | 100 | 35 | 150 | 750 | 5 | 3.214 | 3.214 | 50 |
| 8 | 2mm | 0.10 | 100 | 35 | 175 | 875 | 5 | 4,375 | 4.375 | 50 |
| 9 | 2mm | 0.10 | 100 | 38 | 200 | 1000 | 5 | 5.263 | 5.263 | 50 |
| 10 | 2mm | 0.10 | 100 | 36 | 225 | 1125 | 5 | 7.031 | 7.031 | 50 |
| 11 | 2mm | 0.10 | 100 | 35 | 250 | 1250 | 5 | 8.929 | 8.929 | 50 |

[Table 2-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 μl) total | Calorie strength (J/100 μl) per one pulse | | |
| 1 | - | - | - | - | - | - | - | >95 | 0 |
| 2 | 20 | 100 | 50 | 50 | 10 | 5.714 | 0.571 | >95 | 0 |
| 3 | 20 | 100 | 50 | 50 | 10 | 5.714 | 0.571 | >95 | 0 |
| 4 | 20 | 100 | 50 | 50 | 10 | 5.882 | 0.588 | >95 | 30 |
| 5 | 20 | 100 | 50 | 50 | 10 | 5.556 | 0.556 | 90 | 80 |
| 6 | 20 | 100 | 50 | 50 | 10 | 5.000 | 0.500 | 90 | 92 |
| 7 | 20 | 100 | 50 | 50 | 10 | 5.714 | 0.571 | 80 | 94 |
| 8 | 20 | 100 | 50 | 50 | 10 | 5.714 | 0.571 | 50 | 96 |
| 9 | 20 | 100 | 50 | 50 | 10 | 5.263 | 0.526 | 20 | 87 |
| 10 | 20 | 100 | 50 | 50 | 10 | 5.556 | 0.556 | 0 | 0 |
| 11 | 20 | 100 | 50 | 50 | 10 | 5.714 | 0.571 | 0 | 0 |

<Example 3> Examination of voltage of the second electric pulse (1)

As shown in Tables 3, the second electric pulse was applied by varying its voltage. Other conditions for electroporation were same to Example 1 (samples 13-20).

Note that a sample, which was not subjected to electric pulse treatment after put into the cuvette, was used as a control (sample 12).

And the viability and the gene transferring rate were calculated in the same manner as in Example 1. The result is shown in Tables 3.

The result showed that the second electric pulse applied under a suitable condition can elevate largely the viability.

Specifically, when voltage of the second electric pulse was adjusted so as to be controlled in the range of electric field strength=50-250 V/cm and calorie strength per pulse=0.13-3.57 J/100 $\mu$L, both the viability and the gene transferring rate were as high as 50% or more. Especially in the case of electric field strength=75-125 V/cm and calorie strength per pulse= 0.31-0.68 J/100 $\mu$L, the viability and the gene transferring rate were as high as 80% or more.

[Table 3-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100 $\mu$l) per one pulse | |
| 12 | 2mm | 0.10 | 100 | - | - | - | - | - | - | - |
| 13 | 2mm | 0.10 | 100 | 36 | 125 | 625 | 5 | 2.170 | 2.170 | 50 |
| 14 | 2mm | 0.10 | 100 | 37 | 125 | 625 | 5 | 2.111 | 2.111 | 50 |
| 15 | 2mm | 0.10 | 100 | 36 | 125 | 625 | 5 | 2.170 | 2.170 | 50 |
| 16 | 2mm | 0.10 | 100 | 36 | 125 | 625 | 5 | 2.170 | 2.170 | 50 |
| 17 | 2mm | 0.10 | 100 | 46 | 125 | 625 | 5 | 1.698 | 1.698 | 50 |
| 18 | 2mm | 0.10 | 100 | 36 | 125 | 625 | 5 | 2.170 | 2.170 | 50 |
| 19 | 2mm | 0.10 | 100 | 35 | 125 | 625 | 5 | 2.232 | 2.232 | 50 |
| 20 | 2mm | 0.10 | 100 | 35 | 125 | 625 | 5 | 2.232 | 2.232 | 50 |

[Table 3-B]

| Sample | Second electric pulse | | | | | | | Viability (%) | Gene transferring rate (%) |
|---|---|---|---|---|---|---|---|---|---|
| | Voltage (V) | Electric strength (V/cm) | Pulse length (ms) | Pulse interval (ms) | Number of pulses | Calorie strength (J/100 $\mu l$) total | Calorie strength (J/100 $\mu l$) per one pulse | | |
| 12 | - | - | - | - | - | - | - | >95 | 0 |
| 13 | 5 | 25 | 50 | 50 | 10 | 0.347 | 0.035 | 20 | 66 |
| 14 | 10 | 50 | 50 | 50 | 10 | 1.351 | 0.135 | 80 | 88 |
| 15 | 15 | 75 | 50 | 50 | 10 | 3.125 | 0.313 | 90 | 91 |
| 16 | 20 | 100 | 50 | 50 | 10 | 5.556 | 0.556 | 90 | 91 |
| 17 | 25 | 125 | 50 | 50 | 10 | 6.793 | 0.679 | 80 | 89 |
| 18 | 30 | 150 | 50 | 50 | 10 | 12.500 | 1.250 | 60~70 | 94 |
| 19 | 40 | 200 | 50 | 50 | 10 | 22.857 | 2.286 | 50 | 95 |
| 20 | 50 | 250 | 50 | 50 | 10 | 35.714 | 3.571 | 50 | 92 |

<Example 4> Examination of voltage of the second electric pulse (2)

As shown in Tables 4, the second electric pulse was applied by varying its voltage. Other conditions for electroporation were same to Example 1 (samples 57-60).

Note that a sample, which was not subjected to electric pulse treatment after put into the cuvette, was used as a control (sample 52).

And the viability and the gene transferring rate were calculated in the same manner as in Example 1. The result is shown in Tables 4.

The result showed that the second electric pulse applied under a suitable condition can elevate largely the viability. Specifically, when voltage of the second electric pulse was adjusted so as to be controlled in the range of electric field strength=15-100 V/cm and calorie strength per pulse=0.01-0.59 J/100 $\mu$L, both the viability and the gene transferring rate were as high as 60% or more. Especially in the case of electric field strength=35-100 V/cm and calorie strength per pulse=0.07-0.59 J/100 $\mu$L, the viability and the gene transferring rate were 80% or more.

[Table 4-A]

| Sample | Cuvette | DNA concentration ($\mu g/\mu l$) | Volume ($\mu l$) | Electric impedance ($\Omega$) | First electric pulse | | | | | Pulse interval (fits) |
| | | | | | Voltage (V) | Electric field strength (V/cm) | Pulse length (ms) | Calorie strength (J/100 $\mu l$) total | Calorie strength (J/100 $\mu l$) Per one pulse | |
|---|---|---|---|---|---|---|---|---|---|---|
| 52 | 2mm | 0.10 | 100 | - | - | - | - | - | - | - |
| 57 | 2mm | 0.10 | 100 | 39 | 125 | 625 | 5 | 2.056 | 2.056 | 50 |
| 58 | 2mm | 0.10 | 100 | 37 | 125 | 625 | 5 | 2.111 | 2.111 | 50 |
| 59 | 2mm | 0.10 | 100 | 35 | 125 | 625 | 5 | 2.232 | 2.232 | 50 |
| 60 | 2mm | 0.10 | 100 | 34 | 125 | 625 | 5 | 2.298 | 2.298 | 50 |

[Table 4-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 $\mu$l) total | Calorie strength (J/100 $\mu$l) per one pulse | | |
| 52 | - | - | - | - | - | - | - | >90 | 0 |
| 57 | 3 | 15 | 50 | 50 | 10 | 0.118 | 0.012 | 60 | 83 |
| 58 | 5 | 25 | 50 | 50 | 10 | 0.338 | 0.034 | 60 | 83 |
| 59 | 7 | 35 | 50 | 50 | 10 | 0.700 | 0.070 | 80 | 85 |
| 60 | 20 | 100 | 50 | 50 | 10 | 5.882 | 0.588 | >90 | 86 |

<Example 5> Examination of electric field strength of the first electric pulse (1)

As shown in Tables 5, the first electric pulse was applied by varying its voltage and pulse length so as to keep the calorie strength constant. Other conditions for electroporation were same to Example 1 (samples 28-36).

Note that a sample, which was not subjected to electric pulse treatment after put into the cuvette, was used as a control (sample 27) .

And the viability and the gene transferring rate were calculated in the same manner as in Example 1. The result is shown in Tables 5.

The result showed that gene transferring was never achieved under electric field strength of 250 V/cm or less (less than 357 V/cm), even if the total calorie strength of the first electric pulse was kept almost constant (1.69-2.35 J1100 $\mu$L).

This result suggests that an electric field strength equal to or more than a specific value is necessary to enable the effect of the first electric pulse to be exhibited.

[Table 5-A]

| Sample | Cuvette | DNA concentraction ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100 $\mu$l) per one pulse | |
| 27 | 2mm | 0.10 | 100 | - | - | - | - | - | - | - |
| 28 | 2mm | 0.10 | 100 | 48 | 30 | 150 | 90 | 1.688 | 1.688 | 50 |
| 29 | 2mm | 0.10 | 100 | 34 | 50 | 250 | 35 | 2.574 | 2.574 | 50 |
| 30 | 2mm | 0.10 | 100 | 38 | 50 | 250 | 50 | 3.289 | 3.289 | 50 |
| 31 | 2mm | 0.10 | 100 | 41 | 75 | 375 | 15 | 2.058 | 2.058 | 50 |
| 32 | 2mm | 0.10 | 100 | 34 | 100 | 500 | 8 | 2.353 | 2.353 | 50 |
| 33 | 2mm | 0.10 | 100 | 36 | 125 | 625 | 5 | 2.170 | 2.170 | 50 |
| 34 | 2mm | 0.10 | 100 | 38 | 150 | 750 | 4 | 2.368 | 2.368 | 50 |
| 35 | 2mm | 0.10 | 100 | 33 | 175 | 875 | 3 | 2.784 | 2.784 | 50 |
| 36 | 2mm | 0.10 | 100 | 34 | 200 | 1000 | 2 | 2.353 | 2.353 | 50 |

[Table 5-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 μl) total | Calorie strength (J/100 μl) per one pulse | | |
| 27 | - | - | - | - | - | - | - | >95 | 0 |
| 28 | 20 | 100 | 50 | 50 | 10 | 4.167 | 0.417 | >90 | 0 |
| 29 | 20 | 100 | 50 | 50 | 10 | 5.882 | 0.588 | >90 | 0 |
| 30 | 20 | 100 | 50 | 50 | 10 | 5.263 | 0.526 | >90 | 0 |
| 31 | 20 | 100 | 50 | 50 | 10 | 4.878 | 0.488 | 90 | 45 |
| 32 | 20 | 100 | 50 | 50 | 10 | 5.882 | 0.588 | 90 | 93 |
| 33 | 20 | 100 | 50 | 50 | 10 | 5.556 | 0.556 | 70 | 94 |
| 34 | 20 | 100 | 50 | 50 | 10 | 5.263 | 0.526 | 70 | 96 |
| 35 | 20 | 100 | 50 | 50 | 10 | 6.061 | 0.606 | 50 | 92 |
| 36 | 20 | 100 | 50 | 50 | 10 | 5.882 | 0.588 | 50 | 95 |

<Example 6> Examination of electric field strength of the first electric pulse (2)

As shown in Tables 6, the first electric pulse was applied by varying its voltage and pulse length so as to keep the calorie strength constant. Other conditions for electroporation were same to Example 1 (samples 104-112).

Note that a sample, which was not subjected to electric pulse treatment after put into the cuvette, was used as a control (sample 103).

And the viability and the gene transferring rate were calculated in the same manner as in Example 1. The result is shown in Tables 6.

The result showed that high viability and gene transferring rate were obtained by keeping the calorie strength of the first electric pulse almost constant (1.66-2.08 J/100 $\mu$L), even if high electric field strength of 4,500 V/cm was applied.

This result suggests that the calorie strength, not voltage itself, of the first electric pulse has an impact on the viability.

[Table 6-A]

| Sample | Cuvette | DNA concentration (μg/μl) | Volume (μl) | Electric impedance (Ω) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 μl) total | Calorie strength (J/100 μl) per one pulse | |
| 103 | 2mm | 0.10 | 103 | - | - | - | - | - | - | - |
| 104 | 2mm | 0.10 | 100 | 42 | 100 | 500 | 8 | 1.905 | 1.905 | 50 |
| 105 | 2mm | 0.10 | 100 | 47 | 125 | 625 | 5 | 1.662 | 1.662 | 50 |
| 106 | 2mm | 0.10 | 100 | 41 | 150 | 750 | 3.5 | 1.921 | 1.921 | 50 |
| 107 | 2mm | 0.10 | 103 | 39 | 175 | 875 | 2.5 | 1.963 | 1.963 | 50 |
| 108 | 2mm | 0.10 | 100 | 40 | 200 | 1000 | 2 | 2.000 | 2.000 | 50 |
| 109 | 2mm | 0.10 | 100 | 39 | 300 | 1500 | 0.0 | 2.077 | 2.077 | 50 |
| 110 | 2mm | 0.10 | 100 | 45 | 500 | 2500 | 0.3 | 1.667 | 1.667 | 50 |
| 111 | 2mm | 0.10 | 100 | 41 | 750 | 3750 | 0.15 | 2.058 | 2.058 | 50 |
| 112 | 2mm | 0.10 | 100 | 41 | 900 | 4500 | 0.1 | 1.976 | 1.976 | 50 |

[Table 6-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 μl) total | Calorie strength (J/100 μl) per one pulse | | |
| 103 | - | - | - | - | - | - | - | >95 | 0 |
| 104 | 20 | 100 | 50 | 50 | 10 | 4.762 | 0.476 | 90 | 86 |
| 105 | 20 | 100 | 50 | 50 | 10 | 4.255 | 0.426 | 80 | 95 |
| 106 | 20 | 100 | 50 | 50 | 10 | 4.878 | 0.488 | 70 | 97 |
| 107 | 20 | 100 | 50 | 50 | 10 | 5.128 | 0.513 | 70 | 95 |
| 108 | 20 | 100 | 50 | 50 | 10 | 5.000 | 0.500 | 70 | 95 |
| 109 | 20 | 100 | 50 | 50 | 10 | 5.128 | 0.513 | 70 | 96 |
| 110 | 20 | 100 | 50 | 50 | 10 | 4.444 | 0.444 | 70 | 93 |
| 111 | 20 | 100 | 50 | 50 | 10 | 4.878 | 0.488 | 70 | 96 |
| 112 | 20 | 100 | 50 | 50 | 10 | 4.878 | 0.488 | 80 | 91 |

<Example 7> Examination of pulse length of the first electric pulse

As shown in Tables 7, the first electric pulse was applied by varying its pulse length under the constant electric field strength. Other conditions for electroporation were same to Example 1 (samples 76-78).

And the viability and the gene transferring rate were calculated in the same manner as in Example 1. The result is shown in Tables 7.

The result showed that extremely higher viability and gene transferring rate (both rates=90% or more) were obtained by applying the first electric pulse of short pulse length (=10-20 m sec) under keeping its electric field strength=500 V/cm constant.

But in the case where a pulse length was as long as 30 m sec, decreased viability was observed. It is thought that an increase in calorie strength resulted in the decreased viability.

[Table 7-A]

| Sample | Cuvette | DNA concentration (μg/μl) | Volume (μl) | Electric impedance (Ω) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 μl) total | Calorie strength (J/100 μl) per one pulse | |
| 70 | 2mm | 0.10 | 100 | 40 | 100 | 500 | 10 | 2.500 | 2.500 | 50 |
| 77 | 2mm | 0.10 | 100 | 47 | 100 | 500 | 20 | 4.255 | 4.255 | 50 |
| 78 | 2mm | 0.10 | 100 | 41 | 100 | 500 | 30 | 7.317 | 7.317 | 50 |

[Table 7-B]

**Second electric pulse**

| Sample | Voltage (V) | Electric field strength (V/cm) | Pulse length (ms) | Pulse interval (ms) | Number of pulses | Calorie strength (J/100 μl) total | Calorie strength (J/100 μl) per one pulse | Viability (%) | Gene transferring rate (%) |
|---|---|---|---|---|---|---|---|---|---|
| 76 | 20 | 100 | 50 | 50 | 10 | 5.000 | 0.500 | 90 | 93 |
| 77 | 20 | 100 | 50 | 50 | 10 | 4.255 | 0.426 | 90 | 95 |
| 78 | 20 | 100 | 50 | 50 | 10 | 4.878 | 0.488 | 50 | 91 |

<Example 8> Examination of pulse frequency of the first electric pulse (1)

As shown in Tables 8, the first electric pulse was applied by varying its pulse frequency and pulse length. Other conditions for electroporation were same to Example 1 (samples 71-74).

Note that a sample, which was not subjected to electric pulse treatment after put into the cuvette, was used as a control (sample 70).

And the viability and the gene transferring rate were calculated in the same manner as in Example 1. The result is shown in Tables 8.

The result showed that similar results of the viability and the gene transferring rate were obtained when the first electric pulse was applied so that the total calorie strength was kept constant (samples 71-73).

But the result showed that decreased viability was obtained in the case where the total calorie strength became excessively high by increasing only the frequency (samples 71 and 74).

These results suggest that the total calorie strength, not calorie strength per pulse, of the first electric pulse has an impact on the viability and the gene transferring rate.

[Table 8-A]

| Sample | Sample Cuvette | DNA concentraction ($\mu$g/$\mu$l)) | Volume ($\mu$l) | Electric impedance ($\Omega$) | First electric pulse | | | | | | | Pulse interval (ms) |
| | | | | | Voltage (V) | Electric field strength (V/cm) | Pulse length (ms) | Pulse interval (ms) | Number of pulses | Calorie strength (J/100 $\mu$l) total | Calorie strength (J/100 $\mu$l) per one pulse | |
|---|---|---|---|---|---|---|---|---|---|---|---|---|
| 70 | 2mm | 0.1 | 100 | - | - | - | - | - | - | - | - | - |
| 71 | 2mm | 0.1 | 100 | 41 | 125 | 625 | 5 | 6 | 1 | 1.905 | 1.005 | 50 |
| 72 | 2mm | 0.1 | 100 | 37 | 125 | 625 | 1 | 5 | 5 | 2.111 | 0.422 | 50 |
| 73 | 2mm | 0.1 | 100 | 36 | 125 | 625 | 2.5 | 5 | 2 | 2.170 | 1.005 | 50 |
| 74 | 2mm | 0.1 | 100 | 36 | 125 | 625 | 5 | 5 | 2 | 4.340 | 2.170 | 50 |

[Table 8-B]

| Sample | Second electric pulse | | | | | | | Viability (%) | Gene transferring rate (%) |
| | Voltage (V) | Electric field strength (V/cm) | Pulse length (ms) | Pulse internal (ms) | Number of pulses | Calorie strength (J/100μl) total | Calorie strength (J/100μl) per one pulse | | |
|---|---|---|---|---|---|---|---|---|---|
| 70 | - | - | - | - | - | - | - | >95 | 0 |
| 71 | 20 | 100 | 50 | 50 | 10 | 4.878 | 0.488 | 90 | 94 |
| 72 | 20 | 100 | 50 | 50 | 10 | 5.405 | 0.541 | 90 | 88 |
| 73 | 20 | 100 | 50 | 50 | 10 | 5.556 | 0.556 | 90 | 93 |
| 74 | 20 | 100 | 50 | 50 | 10 | 5.556 | 0.556 | 80 | 94 |

<Example 9> Examination of pulse frequency of the first electric pulse (2)

As shown in Tables 9, the first electric pulse was applied by varying its pulse frequency and pulse length. Other conditions for electroporation were same to Example 1 (samples 179-190).

Note that a sample, which was not subjected to electric pulse treatment after put into the cuvette, was used as a control (sample 178).

And the viability and the gene transferring rate were calculated in the same manner as in Example 1. The result is shown in Tables 9.

The result showed that the gene transferring rate was elevated by increasing the frequency to increase the total calorie strength, even if the calorie strength per pulse was lower (for example, less than 0.2 J/100 μL in samples 182, 184-188).

But it suggested that gene transferring was never achieved under total calorie strength of 0.286 J/100 μL or less, even if the frequency was increased (samples 189 and 190).

[Table 9-A]

| Sample | Cuvette | DNA concentration (μg/μl) | Volume (μl) | Electric impedance (Ω) | First electric pulse | | | | | | | Pulse interval (ms) |
|---|---|---|---|---|---|---|---|---|---|---|---|---|
| | | | | | Voltage (V) | Electric field strength (V/cm) | Pulse length (ms) | Pulse interval (ms) | Number of pulses | Caloric strength (J/100 μl) total | Calorie strength (J/100 μl) per one pulse | |
| 178 | 2mm | 0.1 | 100 | - | - | - | - | - | - | - | - | |
| 179 | 2mm | 0.1 | 100 | 140 | 125 | 625 | 5 | 50 | 1 | 0.558 | 0.558 | 50 |
| 180 | 2mm | 0.1 | 100 | 175 | 125 | 625 | 5 | 50 | 2 | 0.893 | 0.446 | 50 |
| 181 | 2mm | 0.1 | 100 | 102 | 125 | 625 | 5 | 50 | 3 | 2.298 | 0.766 | 50 |
| 182 | 2mm | 0.1 | 100 | 124 | 125 | 625 | 25 | 50 | 2 | 0.630 | 0.315 | 50 |
| 183 | 2mm | 0.1 | 100 | 61 | 125 | 625 | 2.5 | 50 | 3 | 1.921 | 0.640 | 50 |
| 184 | 2mm | 0.1 | 100 | 104 | 125 | 625 | 1 | 50 | 3 | 0.236 | 0.095 | 50 |
| 185 | 2mm | 0.1 | 100 | 92 | 125 | 625 | 1 | 50 | 5 | 0.849 | 0.170 | 50 |
| 186 | 2mm | 0.1 | 100 | 74 | 123 | 625 | 1 | 50 | 10 | 2.111 | 0.211 | 50 |
| 187 | 2mn | 0.1 | 100 | 73 | 125 | 625 | 0.5 | 50 | 5 | 0.535 | 0.107 | 50 |
| 188 | 2mm | 0.1 | 100 | 153 | 125 | 625 | 0.5 | 50 | 10 | 0.511 | 0.051 | 50 |
| 180 | 2mm | 0.1 | 100 | 02 | 125 | 625 | 01 | 50 | 5 | 0.005 | 0.010 | 50 |
| 100 | 2mm | 0.1 | 100 | 105 | 125 | 625 | 01 | 50 | 10 | 0.030 | 0.000 | 50 |

[Table 9-B]

| Sample | Second electric pulse | | | | | | | Viability (%) | Gene transferring rate (%) |
| | Voltage (V) | Electric strength (V/cm) | Pulse length (ms) | Pulse interval (ms) | Number of pulses | Calorie strength (J/100 $\mu$l) total | Calorie strength (J/100 $\mu$l) per one pulse | | |
|---|---|---|---|---|---|---|---|---|---|
| 178 | | | | | | | | >95 | 0 |
| 170 | 20 | 100 | 50 | 50 | 1 | 0.143 | 0.143 | 90 | 72 |
| 180 | 20 | 100 | 50 | 50 | 1 | 0.114 | 0.114 | 80 | 92 |
| 181 | 20 | 100 | 50 | 50 | 1 | 0.190 | 0.190 | 70 | 96 |
| 182 | 20 | 100 | 50 | 50 | 1 | 0.161 | 0.161 | 80 | 76 |
| 183 | 20 | 100 | 50 | 50 | 1 | 0.323 | 0.328 | 80 | 95 |
| 184 | 20 | 100 | 50 | 50 | 1 | 0.122 | 0.122 | 90 | 51 |
| 185 | 20 | 100 | 60 | 50 | 1 | 0.217 | 0.217 | 80 | 87 |
| 186 | 20 | 100 | 50 | 50 | 1 | 0.270 | 0.270 | 70 | 94 |
| 187 | 20 | 100 | 50 | 50 | 1 | 0.274 | 0.274 | 90 | 73 |
| 188 | 20 | 100 | 50 | 50 | 1 | 0.131 | 0.131 | 90 | 45 |
| 189 | 20 | 100 | 50 | 50 | 1 | 0.244 | 0.244 | 90 | <10* |
| 190 | 20 | 100 | 50 | 50 | 1 | 0.103 | 0.103 | 90 | ~0* |

EP 2 527 433 B1

37

<Example 10> Examination of calorie ($V^2I\sqrt{T}$) of the first electric pulse

As shown in Tables 10, the first electric pulse was applied by varying its voltage and pulse length so as to keep $V^2I\sqrt{T}$ (calorie value) constant. Other conditions for electroporation were same to Example 1 (samples 62-67).

Note that a sample, which was not subjected to electric pulse treatment after put into the cuvette, was used as a control (sample 61).

And the viability and the gene transferring rate were calculated in the same manner as in Example 1. The result is shown in Tables 10.

The result showed that the viability and the gene transferring rate had no correlation with $V^2I\sqrt{IT}$ (calorie value) of the first electric pulse but depended upon its calorie strength (J/100 $\mu$L) and electric field strength (V/cm).

[Table 10-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 61 | 2mm | 0.10 | 100 | - | - | - | - | - | - | - |
| 62 | 2mm | 0.10 | 100 | 35 | 75 | 375 | 99.9 | 16.055 | 16.055 | 50 |
| 63 | 2mm | 0.10 | 100 | 34 | 100 | 500 | 15 | 4.412 | 4.412 | 50 |
| 64 | 2mm | 0.10 | 100 | 37 | 125 | 625 | 5 | 2.111 | 2.111 | 50 |
| 65 | 2mm | 0.10 | 100 | 37 | 150 | 750 | 2 | 1.216 | 1.216 | 50 |
| 66 | 2mm | 0.10 | 100 | 35 | 175 | 875 | 0.5 | 0.438 | 0.438 | 50 |
| 67 | 2mm | 0.10 | 100 | 35 | 200 | 1000 | 0.3 | 0.343 | 0.343 | 50 |

[Table 10-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 $\mu$l) total | Calorie strength (J1/00 $\mu$l) per one pulse | | |
| 61 | - | - | - | - | - | - | - | >95 | 0 |
| 62 | 20 | 100 | 50 | 50 | 10 | 5.714 | 0.571 | >90 | 49 |
| 63 | 20 | 100 | 50 | 50 | 10 | 5.882 | 0.588 | 90~90 | 91 |
| 64 | 20 | 100 | 50 | 50 | 10 | 5.405 | 0.541 | 80 | 76 |
| 65 | 20 | 100 | 50 | 50 | 10 | 5.405 | 0.541 | 80~90 | 85 |
| 68 | 20 | 100 | 50 | 50 | 10 | 5.714 | 0.571 | 80~90 | 63 |
| 67 | 20 | 100 | 50 | 50 | 10 | 5.714 | 0.571 | 80 | 61 |

<Example 11> Examination of calorie ($V^2IT$) of the first electric pulse

As shown in Tables 11, the first electric pulse was applied by varying its voltage and pulse length so as to keep a $V^2IT$ value (calorie value) constant. Other conditions for electroporation were same to Example 1 (samples 114-122).

Note that a sample, which was not subjected to electric pulse treatment after put into the cuvette, was used as a control (sample 113).

And the viability and the gene transferring rate were calculated in the same manner as in Example 1. The result is shown in Tables 11.

The result showed that the viability and the gene transferring rate had no correlation with $V^2IT$ (calorie value) of the first electric pulse but depended upon its calorie strength (J/100 μL).

And the result showed also that the gene transferring rate would drop down largely when the calorie strength dropped down under ca. 0.28 J/100 μL.

[Table 11-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100 $\mu$l) per one pulse | |
| 113 | 2mm | 0.10 | 100 | - | - | - | - | - | - | - |
| 114 | 2mm | 0.10 | 100 | 44 | 100 | 500 | 10 | 2.273 | 2.273 | 50 |
| 115 | 2mm | 0.10 | 100 | 49 | 125 | 625 | 5 | 1.594 | 1.594 | 50 |
| 116 | 2mm | 0.10 | 100 | 41 | 150 | 750 | 3 | 1.646 | 1.646 | 50 |
| 117 | 2mm | 0.10 | 100 | 41 | 175 | 875 | 1.8 | 1.345 | 1.345 | 50 |
| 118 | 2mm | 0.10 | 100 | 40 | 200 | 1000 | 1.2 | 1.200 | 1.200 | 50 |
| 119 | 2mm | 0.10 | 100 | 39 | 300 | 1500 | 0.35 | 0.808 | 0.808 | 50 |
| 120 | 2mm | 0.10 | 100 | 42 | 500 | 2500 | 0.08 | 0.476 | 0.476 | 50 |
| 121 | 2mm | 0.10 | 100 | 40 | 750 | 3750 | 0.02 | 0.281 | 0.281 | 50 |
| 122 | 2mm | 0.10 | 100 | 38 | 900 | 4500 | 0.01 | 0.213 | 0.213 | 50 |

EP 2 527 433 B1

[Table 11-B]

| Sample | Second electric pulse | | | | | | | Viability (%) | Gene transferring rate (%) |
|---|---|---|---|---|---|---|---|---|---|
| | Voltage (V) | Electric strength (V/cm) | Pulse length (ms) | Pulse interval (ms) | Number of pulses | Calorie strength (J/100 μl) total | Calorie strength (J/100 μl) per one pulse | | |
| 113 | - | - | - | - | - | - | - | >95 | 0 |
| 114 | 20 | 100 | 50 | 50 | 10 | 4.545 | 0.455 | >95 | 85 |
| 115 | 20 | 100 | 50 | 50 | 10 | 4.082 | 0.408 | 90 | 90 |
| 116 | 20 | 100 | 50 | 50 | 10 | 4.878 | 0.488 | 80 | 93 |
| 117 | 20 | 100 | 50 | 50 | 10 | 4.878 | 0.488 | 70 | 91 |
| 118 | 20 | 100 | 50 | 50 | 10 | 5.000 | 0.500 | 70 | 88 |
| 119 | 20 | 100 | 50 | 50 | 10 | 5.128 | 0.513 | 80 | 87 |
| 120 | 20 | 100 | 50 | 50 | 10 | 4.762 | 0.476 | 80 | 71 |
| 121 | 20 | 100 | 50 | 50 | 10 | 5.000 | 0.500 | 80 | 37 |
| 122 | 20 | 100 | 50 | 50 | 10 | 5.263 | 0.526 | 90 | 19 |

<Example 12> Examination of calorie (VI√T) of the first electric pulse

As shown in Tables 12, the first electric pulse was applied by varying its voltage and pulse length so as to keep its calorie strength constant. Other conditions for electroporation were same to Example 1 (samples 124-131).

Note that a sample, which was not subjected to electric pulse treatment after put into the cuvette, was used as a control (sample 123).

And the viability and gene transferring rate were calculated in the same manner as in Example 1. The result is shown in Tables 12.

The result showed that the viability and the gene transferring rate had no correlation with VI√T (calorie value) of the first electric pulse but depended upon calorie strength (J/100 $\mu$L).

And the result showed also that the gene transferring rate would drop down largely when the calorie strength dropped down under ca. 0.34 J/100 $\mu$L.

[Table 12-A]

| Sample | Cuvette | DNA concentration ($\mu g/\mu l$) | Volume ($\mu l$) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu l$) total | Calorie strength (J/100$\mu l$) per one pulse | |
| 123 | 2mm | 0.10 | 100 | - | - | - | - | - | - | - |
| 124 | 2mm | 0.10 | 100 | 34 | 100 | 500 | 15 | 4.412 | 4.412 | 50 |
| 125 | 2mm | 0.10 | 100 | 35 | 125 | 625 | 5 | 2.232 | 2.232 | 50 |
| 126 | 2mm | 0.10 | 100 | 36 | 150 | 750 | 2.5 | 1.563 | 1.563 | 50 |
| 127 | 2mm | 0.10 | 100 | 49 | 175 | 875 | 1.5 | 0.938 | 0.938 | 50 |
| 128 | 2mm | 0.10 | 100 | 38 | 200 | 1000 | 1 | 1.053 | 1.053 | 50 |
| 129 | 2mm | 0.10 | 100 | 40 | 300 | 1500 | 0.15 | 0.338 | 0.338 | 50 |
| 130 | 2mm | 0.10 | 100 | 39 | 500 | 2500 | 0.02 | 0.128 | 0.123 | 50 |
| 131 | 2mm | 0.10 | 100 | 54 | 600 | 3000 | 0.01 | 0.067 | 0.067 | 50 |

[Table 12-B]

| Sample | Second electric pulse | | | | | | | Viability (%) | Gene transferring rate (%) |
| | Voltage (V) | Electric field strength (V/cm) | Pulse length (ms) | Pulse interval (ms) | Number of pulses | Calorie strength (J/100$\mu$l) total | Calorie strength (J/100$\mu$l) per one pulse | | |
| --- | --- | --- | --- | --- | --- | --- | --- | --- | --- |
| 123 | - | - | - | - | - | - | - | >95 | 0 |
| 124 | 20 | 100 | 50 | 50 | 10 | 5.882 | 0.588 | 90 | 90 |
| 125 | 20 | 100 | 50 | 50 | 10 | 5.714 | 0.571 | 90 | 95 |
| 126 | 20 | 100 | 50 | 50 | 10 | 5.556 | 0.558 | 80 | 94 |
| 127 | 20 | 100 | 50 | 50 | 10 | 4.082 | 0.408 | 80 | 91 |
| 128 | 20 | 100 | 50 | 50 | 10 | 5.263 | 0.526 | 80 | 87 |
| 129 | 20 | 100 | 50 | 50 | 10 | 5.000 | 0.500 | 70 | 40 |
| 130 | 20 | 100 | 50 | 50 | 10 | 5.128 | 0.513 | 80 | 18 |
| 131 | 20 | 100 | 50 | 50 | 10 | 3.704 | 0.370 | 90 | 8 |

<Example 13> Examination of pulse frequency of the second electric pulse

As shown in Tables 13, the second electric pulse was applied by varying its pulse frequency. Other conditions for electroporation were same to Example 1 (samples 22-26).

Note that a sample, which was not subjected to electric pulse treatment after put into the cuvette, was used as a control (sample 21).

And the viability and the gene transferring rate were calculated in the same manner as in Example 1. The result is shown in Tables 13.

The result showed that repeated application of the second electric pulse (more than three times, optimally 10 times) was able to elevate the viability further.

[Table 13-A]

| Sample | Cuvette | DNA concentraction ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 21 | 2mm | 0.10 | 100 | - | - | - | - | - | - | - |
| 22 | 2mm | 0.10 | 100 | 36 | 125 | 625 | 5 | 2.170 | 2.170 | 50 |
| 23 | 2mm | 0.10 | 100 | 35 | 125 | 625 | 5 | 2.232 | 2.232 | 50 |
| 24 | 2mm | 0.10 | 100 | 35 | 125 | 625 | 5 | 2.232 | 2.232 | 50 |
| 25 | 2mm | 0.10 | 100 | 36 | 125 | 625 | 5 | 2.170 | 2.170 | 50 |
| 26 | 2mm | 0.10 | 100 | 36 | 125 | 625 | 5 | 2.170 | 2.170 | 50 |

[Table 13-B]

| Sample | Second electric pulse | | | | | | | Viability (%) | Gene transferring rate (%) |
| | Voltage (V) | Electric field strength (V/cm) | Pulse length (ms) | Pulse interval (ms) | Number of pulses | Calorie strength (J/100 $\mu$l) total | Calorie strength (J/100/$\mu$l) per one pulse | | |
|---|---|---|---|---|---|---|---|---|---|
| 21 | - | - | - | - | - | - | - | >95 | 0 |
| 22 | 20 | 100 | 50 | 50 | 1 | 0.556 | 0.558 | 80 | 91 |
| 23 | 20 | 100 | 50 | 50 | 3 | 1.714 | 0.571 | 90 | 90 |
| 24 | 20 | 100 | 50 | 50 | 5 | 2.357 | 0.571 | 90 | 90 |
| 25 | 20 | 100 | 50 | 50 | 7 | 3.889 | 0.556 | 85 | 88 |
| 26 | 20 | 100 | 50 | 50 | 10 | 5.556 | 0.556 | 95 | 91 |

<Example 14> Examination of pulse interval between the first electric pulse and the second electric pulse (1)

As shown in Tables 14, electric pulses were applied by varying the pulse interval between the first electric pulse and the second electric pulse. Other conditions for electroporation were same to Example 1 (samples 38-44).

Note that a sample, which was not subjected to electric pulse treatment after put into the cuvette, was used as a control (sample 37).

And the viability and the gene transferring rate were calculated in the same manner as in Example 1. The result is shown in Tables 14.

The result showed that a shorter pulse interval tended to result in higher viability and a longer pulse interval tended to result in lower viability, and that the viability was elevated largely especially by setting the pulse interval to 99.9 m sec (about 0.1 sec) or less.

But the result suggested that comparatively higher viability was obtained even under as a long pulse interval as one minute.

[Table 14-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 37 | 2mm | 0.10 | 100 | - | - | - | - | - | - | - |
| 38 | 2mm | 0.10 | 100 | 34 | 125 | 625 | 5 | 2.298 | 2.293 | 5 msec |
| 39 | 2mm | 0.10 | 100 | 32 | 125 | 625 | 5 | 2.441 | 2.441 | 50 msec |
| 40 | 2mm | 0.10 | 100 | 34 | 125 | 625 | 5 | 2.298 | 2.293 | 99.9 msec |
| 41 | 2mm | 0.10 | 100 | 33 | 125 | 625 | 5 | 2.367 | 2.367 | 5 sec |
| 42 | 2mm | 0.10 | 100 | 33 | 125 | 625 | 5 | 2.367 | 2.367 | 10 sec |
| 43 | 2mm | 0.10 | 100 | 34 | 125 | 625 | 5 | 2.298 | 2.298 | 1 min |
| 44 | 2mm | 0.10 | 100 | 36 | 125 | 625 | 5 | 2.170 | 2.170 | 10 min |

[Table 14-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse internal (ms) | Number of Pulses | Calorie strength (J/100µl) total | Calorie strength (J/100 µl) per one pulse | | |
| 37 | - | - | - | - | - | - | - | >95 | 0 |
| 38 | 20 | 100 | 50 | 50 | 10 | 5.882 | 0.588 | 80 | 93 |
| 39 | 20 | 100 | 50 | 50 | 10 | 6.250 | 0.625 | 80 | 93 |
| 40 | 20 | 100 | 50 | 50 | 10 | 5.882 | 0.588 | 80 | 91 |
| 41 | 20 | 100 | 50 | 50 | 10 | 6.061 | 0.606 | 50 | 91 |
| 42 | 20 | 100 | 50 | 50 | 10 | 6.061 | 0.606 | 70 | 93 |
| 43 | 20 | 100 | 50 | 50 | 10 | 5.882 | 0.588 | 50 | 38 |
| 44 | 20 | 100 | 50 | 50 | 10 | 5.556 | 0.556 | 40 | 88 |

<Example 15> Examination of pulse interval between the first electric pulse and the second electric pulse (2)

As shown in Tables 15, electric pulses were applied by varying the pulse interval between the first electric pulse and the second electric pulse. Other conditions for electroporation were same to Example 1 (samples 46-51).

Note that a sample, which was not subjected to electric pulse treatment after put into the cuvette, was used as a control (sample 45).

And the viability and the gene transferring rate were calculated in the same manner as in Example 1. The result is shown in Tables 15.

The result showed that a shorter pulse interval tended to result in higher viability and a longer pulse interval tended to result in lower viability, and that the viability was elevated largely especially by setting the pulse interval to 99.9 m sec (about 0.1. sec) or less.

But the result suggested that comparatively higher viability was obtained even under as a long pulse interval as one minute.

[Table 15-A]

| Sample | Cuvette | DNA concen-tration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 45 | 2mm | 0.10 | 100 | - | - | - | - | - | - | - |
| 46 | 2mm | 0.10 | 100 | 36 | 125 | 625 | 5 | 2.170 | 2.170 | 5 msec |
| 47 | 2mm | 0.10 | 100 | 38 | 125 | 625 | 5 | 2.056 | 2.056 | 50 msec |
| 48 | 2mm | 0.10 | 100 | 38 | 125 | 625 | 5 | 2.056 | 2.056 | 99.9 msec |
| 49 | 2mm | 0.10 | 100 | 37 | 125 | 625 | 5 | 2.111 | 2.111 | 10 see |
| 50 | 2mm | 0.10 | 100 | 40 | 125 | 625 | 5 | 1.953 | 1.953 | 1 min |
| 51 | 2mm | 0.10 | 100 | 36 | 125 | 625 | 5 | 2.170 | 2.170 | 10 min |

[Table 15-B]

**Second electric pulse**

| Sample | Voltage (V) | Electric field strength (V/cm) | Pulse length (ms) | Pulse interval (ms) | Number of pulses | Calorie strength (J/100μl) total | Calorie strength (J/100μl) per one Pulse | Viability (%) | Gene transfer-ring rate (%) |
|---|---|---|---|---|---|---|---|---|---|
| 45 | - | - | - | - | - | - | - | >95 | 0 |
| 46 | 20 | 100 | 50 | 50 | 10 | 5.556 | 0.556 | 90 | 88 |
| 47 | 20 | 100 | 50 | 50 | 10 | 5.263 | 0.526 | 90 | 87 |
| 48 | 20 | 100 | 50 | 50 | 10 | 5.263 | 0.526 | 90 | 85 |
| 49 | 20 | 100 | 50 | 50 | 10 | 5.405 | 0.541 | 70 | 81 |
| 50 | 20 | 100 | 50 | 50 | 10 | 5.000 | 0.500 | 60 | 79 |
| 51 | 20 | 100 | 50 | 50 | 10 | 5.556 | 0.556 | 40 | 82 |

<Example 16> Examination of buffer volume (1)

As shown in Tables 16, electroporation was done by varying the buffer volume. Other conditions for electroporation were same to Example 1 (samples 85-92).

Note that a sample, which was not subjected to electric pulse treatment after put into the cuvette, was used as a control (sample 84).

And the viability and the gene transferring rate were calculated in the same manner as in Example 1. The result is shown in Tables 16.

The result showed that the electroporating conditions (electric field strength and calorie strength) at 100 $\mu$L were also applicable to the case of buffer volume=100-400 $\mu$L.

[Table 16-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 84 | 2mm | 0.10 | 100 | - | - | - | - | - | - | - |
| 85 | 2mm | 0.10 | 100 | 38 | 125 | 625 | 5 | 2.056 | 2.056 | 50 |
| 86 | 2mm | 0.10 | 200 | 21 | 60 | 300 | 5 | 0.429 | 0.429 | 50 |
| 87 | 2mm | 0.10 | 200 | 20 | 90 | 450 | 5 | 1.013 | 1.013 | 50 |
| 88 | 2mm | 0.10 | 200 | 25 | 125 | 625 | 5 | 1.563 | 1.563 | 50 |
| 80 | 2mm | 0.10 | 400 | 12 | 30 | 150 | 5 | 0.094 | 0.094 | 50 |
| 90 | 2mm | 0.10 | 100 | 16 | 60 | 300 | 5 | 0.281 | 0.281 | 50 |
| 91 | 2mm | 0.10 | 400 | 12 | 90 | 450 | 5 | 0.344 | 0.844 | 50 |
| 92 | 2mm | 0.10 | 400 | 15 | 125 | 025 | 5 | 1.302 | 1.302 | 50 |

[Table 16-B]

| Sample | Second electric pulse | | | | | | | Viability (%) | Gene transferring rate (%) |
| | Voltage (V) | Electric field strength (V/cm) | Pulse length (ms) | Pulse interval (ms) | Number of pulses | Calorie strength (J/100$\mu$l) total | Calorie strength (J/100$\mu$l) per one pulse | | |
|---|---|---|---|---|---|---|---|---|---|
| 84 | - | - | - | - | - | - | - | >95 | 0 |
| 85 | 20 | 100 | 50 | 50 | 10 | 5.263 | 0.526 | >90 | 94 |
| 86 | 10 | 50 | 50 | 50 | 10 | 1.190 | 0.119 | >95 | 0 |
| 87 | 15 | 75 | 50 | 50 | 10 | 2.813 | 0.281 | >90 | 33 |
| 88 | 20 | 100 | 50 | 50 | 10 | 4.000 | 0.400 | 90 | 90 |
| 89 | 5 | 25 | 50 | 50 | 10 | 0.260 | 0.026 | >95 | 0 |
| 90 | 10 | 50 | 50 | 50 | 10 | 0.781 | 0.078 | >96 | 0 |
| 91 | 15 | 75 | 50 | 50 | 10 | 2.344 | 0.234 | >97 | 14 |
| 92 | 20 | 100 | 50 | 50 | 10 | 3.333 | 0.333 | >98 | 88 |

<Example 17> Examination of buffer volume (2)

As shown in Tables 17, electroporation was done by varying the buffer volume. Other conditions for electroporation were same to Example 1 (samples 147-151).

Note that a sample, which was not subjected to electric pulse treatment after put into the cuvette, was used as a control (sample 146).

And the viability and the gene transferring rate were calculated in the same manner as in Example 1. The result is shown in Tables 17.

The result showed that the electroporating conditions (electric field strength and calorie strength) at 100 μL were also applicable to the case of buffer volume=50-400 μL.

[Table 17-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 146 | 2mm | 0.10 | 100 | - | - | - | - | - | - | - |
| 147 | 2mm | 0.10 | 50 | 81 | 125 | 625 | 5 | 1.929 | 1.929 | 50 |
| 148 | 2mm | 0.10 | 100 | 44 | 125 | 625 | 5 | 1.776 | 1.775 | 50 |
| 149 | 2mm | 0.10 | 200 | 21 | 125 | 625 | 5 | 1.860 | 1.860 | 50 |
| 150 | 2mm | 0.10 | 300 | 17 | 125 | 625 | 5 | 1.532 | 1.532 | 50 |
| 151 | 2mm | 0.10 | 400 | 15 | 125 | 625 | 5 | 1.302 | 1.302 | 50 |

EP 2 527 433 B1

[Table 17-B]

**Second electric pulse**

| Sample | Voltage (V) | Electric field (strength (V/cm) | Pulse length (ms) | Pulse interval (ms) | Number of pulses | Calorie strength (J/100μl) total | Calorie strength (J/100μl) per one pulse | Viability (%) | Gene transferring rate (%) |
|---|---|---|---|---|---|---|---|---|---|
| 148 | - | - | - | - | - | - | - | >95 | 0 |
| 147 | 20 | 100 | 50 | 50 | 10 | 4.938 | 0.494 | 80 | 84 |
| 148 | 20 | 100 | 50 | 50 | 10 | 4.545 | 0.455 | 90 | 86 |
| 149 | 20 | 100 | 50 | 50 | 10 | 4.762 | 0.476 | 90 | 87 |
| 150 | 20 | 100 | 50 | 50 | 10 | 3.922 | 0.392 | >90 | 86 |
| 151 | 20 | 100 | 50 | 50 | 10 | 3.333 | 0.333 | >90 | 83 |

<Example 18> Examination by using 1 mm gap cuvette (1)

As shown in Tables 18, electroporation was done by varying the voltage by using 1 mm gap cuvette. Other conditions for electroporation were same to Example 1 (samples 80-83).

Note that a sample, which was not subjected to electric pulse treatment after put into the cuvette, was used as a control (sample 79).

And the viability and the gene transferring rate were calculated in the same manner as in Example 1. The result is shown in Tables 18.

The result showed that the conditions of electric field strength and calorie strength in the case of using 2 mm gap cuvette were also applicable to the case of using 1 mm gap cuvette.

[Table 18-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 79 | 1mm | 0.10 | 50 | - | - | - | - | - | - | - |
| 80 | 1mm | 0.10 | 50 | 30 | 30 | 300 | 5 | 0.300 | 0.300 | 50 |
| 81 | 1mm | 0.10 | 50 | 29 | 60 | 600 | 5 | 1.241 | 1.241 | 50 |
| 82 | 1mm | 0.10 | 50 | 34 | 90 | 900 | 5 | 2.382 | 2.382 | 50 |
| 83 | 1mm | 0.10 | 50 | 40 | 125 | 1250 | 5 | 3.906 | 3.906 | 50 |

[Table 18-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100$\mu$l) total | Calorie strength (J/100$\mu$l) per one pulse | | |
| 79 | - | - | - | - | - | - | - | >95 | 0 |
| 80 | 5 | 50 | 50 | 50 | 10 | 0.833 | 0.083 | 90 | 0 |
| 81 | 10 | 100 | 50 | 50 | 10 | 3.448 | 0.345 | 70 | 74 |
| 82 | 15 | 150 | 50 | 50 | 10 | 6.618 | 0.662 | 50~60 | 89 |
| 83 | 20 | 200 | 50 | 50 | 10 | 10.000 | 1.000 | 10 | 83 |

<Example 19> Examination by using 1 mm gap cuvette (2)

As shown in Tables 19, electroporation was done by varying the voltage by using 1 mm gap cuvette. Other conditions for electroporation were same to Example 1 (samples 133-143).

Note that a sample, which was not subjected to electric pulse treatment after put into the cuvette, was used as a control (sample 132).

And the viability and the gene transferring rate were calculated in the same manner as in Example 1. The result is shown in Tables 19.

The result showed that the conditions of electric field strength and calorie strength in the case of using 2 mm gap cuvette were also applicable to the case of using 1 mm gap cuvette.

[Table 19-A]

| Sample | Cuvette | DNA concentration ($\mu$g/$\mu$l) | Volume ($\mu$l) | Electric impedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100$\mu$l) total | Calorie strength (J/100$\mu$l) per one pulse | |
| 132 | 1mm | 0.10 | 50 | - | - | - | - | - | - | - |
| 133 | 1mm | 0.10 | 50 | 26 | 60 | 600 | 5 | 1.385 | 1.385 | 50 |
| 134 | 1mm | 0.10 | 50 | 35 | 60 | 600 | 5 | 1.029 | 1.029 | 50 |
| 135 | 1mm | 0.10 | 50 | 33 | 70 | 700 | 5 | 1.485 | 1.485 | 50 |
| 136 | 1mm | 0.10 | 50 | 35 | 70 | 700 | 5 | 1.400 | 1.400 | 50 |
| 137 | 1mm | 0.10 | 50 | 34 | 70 | 700 | 5 | 1.441 | 1.441 | 50 |
| 138 | 1mm | 0.10 | 50 | 34 | 80 | 800 | 5 | 1.882 | 1.882 | 50 |
| 139 | 1mm | 0.10 | 50 | 27 | 80 | 800 | 5 | 2.370 | 2.370 | 50 |
| 140 | 1mm | 0.10 | 50 | 33 | 80 | 800 | 5 | 1.939 | 1.939 | 50 |
| 141 | 1mm | 0.10 | 50 | 29 | 90 | 900 | 5 | 2.793 | 2.793 | 50 |
| 142 | 1mm | 0.10 | 50 | 37 | 90 | 900 | 5 | 2.189 | 2.189 | 50 |
| 143 | 1mm | 0.10 | 50 | 34 | 100 | 1000 | 5 | 2.941 | 2.941 | 50 |

EP 2 527 433 B1

[Table 19-B]

| Sample | Second electric pulse | | | | | | | Viability (%) | Gene transfer-ring rate (%) |
| | Voltage (V) | Electric field strength (V/cm) | Pulse length (ms) | Pulse internal (ms) | Number of pulses | Calorie strength (J/100$\mu$l) total | Calorie strength (J/100$\mu$l) per one pulse | | |
|---|---|---|---|---|---|---|---|---|---|
| 132 | - | - | - | - | - | - | - | >95 | 0 |
| 133 | 5 | 50 | 50 | 50 | 10 | 0.962 | 0.096 | 80 | 41 |
| 134 | 15 | 150 | 50 | 50 | 10 | 6.429 | 0.643 | 80 | 39 |
| 135 | 5 | 50 | 50 | 50 | 10 | 0.758 | 0.076 | 50 | 67 |
| 136 | 10 | 100 | 50 | 50 | 10 | 2.857 | 0.286 | 80 | 74 |
| 137 | 15 | 150 | 50 | 50 | 10 | 6.618 | 0.662 | 60 | 72 |
| 138 | 5 | 50 | 50 | 50 | 10 | 0.735 | 0.074 | 50 | 59 |
| 139 | 10 | 100 | 50 | 50 | 10 | 3.704 | 0.370 | 50 | 83 |
| 140 | 15 | 150 | 50 | 50 | 10 | 6.818 | 0.682 | 50 | 85 |
| 141 | 5 | 50 | 50 | 50 | 10 | 0.862 | 0.086 | 30 | 75 |
| 142 | 10 | 100 | 50 | 50 | 10 | 2.703 | 0.270 | 50 | 85 |
| 143 | 10 | 100 | 50 | 50 | 10 | 2.941 | 0.294 | 50 | 85 |

<Example 20> Examination by using 4 mm gap cuvette (1)

As shown in Tables 20, electroporation was done by varying the voltage by using 4 mm gap cuvette. Other conditions for electroporation were same to Example 1 (samples 154-159).

Note that a sample, which was not subjected to electric pulse treatment after put into the cuvette, was used as a control (sample 153).

And the viability and the gene transferring rate were calculated in the same manner as in Example 1. The result is shown in Tables 20.

The result showed that the conditions of electric field strength and calorie strength in the case of using 2 mm gap cuvette were also applicable to the case of using 4 mm gap cuvette by varying the voltage.

[Table 20-A]

| Sample | Cuvette | DNA concentration ($\mu g/\mu l$) | Volume ($\mu l$) | Electric impedance ($\Omega$) | First electric pulse | | | | | |
|---|---|---|---|---|---|---|---|---|---|---|
| | | | | | Voltage (V) | Electric field strength (V/cm) | Pulse length (ms) | Calorie strength (J/100$\mu l$) total | Calorie strength (J/100$\mu l$) per one pulse | Pulse interval (ms) |
| 153 | 4mm | 0.10 | 200 | - | - | - | - | - | - | - |
| 154 | 4mm | 0.10 | 200 | 68 | 230 | 575 | 5 | 1.945 | 1.945 | 50 |
| 155 | 4mm | 0.10 | 200 | 70 | 270 | 675 | 5 | 2.604 | 2.604 | 50 |
| 156 | 4mm | 0.10 | 200 | 73 | 290 | 725 | 5 | 2.880 | 2.880 | 50 |
| 157 | 4mm | 0.10 | 200 | 73 | 310 | 775 | 5 | 3.080 | 3.080 | 50 |
| 158 | 4mm | 0.10 | 200 | 72 | 330 | 825 | 5 | 3.781 | 3.781 | 50 |
| 159 | 4mm | 0.10 | 200 | 75 | 350 | 875 | 5 | 4.083 | 4.083 | 50 |

[Table 20-B]

| Sample | Second electric pulse | | | | | | | Viability (%) | Gene transferring rate |
|---|---|---|---|---|---|---|---|---|---|
| | voltage (V) | Electric field strength (V/cm) | Pulse length (ms) | Pulse interval (ms) | Number of pulses | Calorie strength (J/100$\mu$l) total | Calorie strength (J/100$\mu$l) per one pulse | | |
| 153 | - | - | - | - | - | - | - | >90 | 0 |
| 154 | 40 | 100 | 50 | 50 | 10 | 5.882 | 0.588 | 90 | 88 |
| 155 | 40 | 100 | 50 | 50 | 10 | 5.714 | 0.571 | 80 | 91 |
| 156 | 40 | 100 | 50 | 50 | 10 | 5.479 | 0.548 | 70 | 92 |
| 157 | 40 | 100 | 50 | 50 | 10 | 5.128 | 0.513 | 60 | 93 |
| 158 | 40 | 100 | 50 | 50 | 10 | 5.556 | 0.556 | 50 | 92 |
| 159 | 40 | 100 | 50 | 50 | 10 | 5.333 | 0.533 | 40 | 93 |

<Example 21> Examination by using 4 mm gap cuvette (2)

As shown in Tables 21, electroporation was done by varying the voltage by using 4 mm gap cuvette. Other conditions for electroporation were same to Example 1 (samples 161-168).

Note that a sample, which was not subjected to electric pulse treatment after put into the cuvette, was used as a control (sample 160).

And the viability and the gene transferring rate were calculated in the same manner as in Example 1. The result is shown in Tables 21.

The result showed that the conditions of electric field strength and calorie strength in the case of using 2 mm gap cuvette were also applicable to the case of using 4 mm gap cuvette by varying the voltage.

[Table 21-A]

| Sample | Cuvette | DNA concentration (μg/μl) | Volume (μl) | Electric impedance (Ω) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100μl) total | Calorie strength (J/100 μl) per one pulse | |
| 160 | 4mm | 0.10 | 200 | - | - | - | - | - | - | - |
| 161 | 4mm | 0.10 | 200 | 79 | 200 | 500 | 5 | 1.266 | 1.266 | 50 |
| 162 | 4mm | 0.10 | 200 | 72 | 210 | 525 | 5 | 1.531 | 1.531 | 50 |
| 163 | 4mm | 0.10 | 200 | 74 | 220 | 550 | 5 | 1.635 | 1.635 | 50 |
| 164 | 4mm | 0.10 | 200 | 76 | 230 | 575 | 5 | 1.740 | 1.740 | 50 |
| 165 | 4mm | 0.10 | 200 | 75 | 240 | 600 | 5 | 1.920 | 1.920 | 50 |
| 166 | 4mm | 0.10 | 200 | 68 | 250 | 625 | 5 | 2.298 | 2.298 | 50 |
| 167 | 4mm | 0.10 | 200 | 72 | 260 | 650 | 5 | 2.347 | 2.347 | 50 |
| 168 | 4mm | 0.10 | 200 | 72 | 270 | 675 | 5 | 2.531 | 2.531 | 50 |

[Table 21-B]

**Second electric pulse**

| Sample | Voltage (V) | Electric field strength (V/cm) | Pulse length (ms) | Pulse interval (ms) | Number of pulses | Calorie strength (J/100μl) total | Calorie strength (J/100 μl) per one pulse | Viability (%) | Gene transferring rate (%) |
|---|---|---|---|---|---|---|---|---|---|
| 160 | - | - | - | - | - | - | - | >95 | 0 |
| 161 | 40 | 100 | 50 | 50 | 10 | 5.063 | 0.506 | >90 | 83 |
| 162 | 40 | 100 | 50 | 50 | 10 | 5.556 | 0.556 | >90 | 90 |
| 163 | 40 | 100 | 50 | 50 | 10 | 5.405 | 0.541 | 80 | 90 |
| 164 | 40 | 100 | 50 | 50 | 10 | 5.263 | 0.526 | 70 | 91 |
| 165 | 40 | 100 | 50 | 50 | 10 | 5.333 | 0.533 | 70 | 91 |
| 166 | 40 | 100 | 50 | 50 | 10 | 5.882 | 0.588 | 70 | 94 |
| 167 | 40 | 100 | 50 | 50 | 10 | 5.556 | 0.556 | 70 | 92 |
| 168 | 40 | 100 | 50 | 50 | 10 | 5.556 | 0.556 | 70 | 93 |

<Example 22> Examination by using 4 mm gap cuvette (3)

As shown in Tables 22, electroporation was done by varying the buffer volume by using 4 mm gap cuvette. Other conditions for electroporation were same to Example 1 (samples 94-102).

Note that a sample, which was not subjected to electric pulse treatment after put into the cuvette, was used as a control (sample 93).

And the viability and the gene transferring rate were calculated in the same manner as in Example 1. The result is shown in Tables 22.

The result showed that the electroporating conditions of electric field strength and calorie strength at 100 $\mu$L in the case of using 2 mm gap cuvette were also applicable to the case where 4 mm gap cuvette was used and buffer volume was 200-800 $\mu$L.

[Table 22-A]

| Sample | Cuvette | DNA concentration (μg/μl) | Volume (μl) | Electric impedance (Ω) | First electric pulse | | | | | Pulse interval (ms) |
|---|---|---|---|---|---|---|---|---|---|---|
| | | | | | Vol Stage (V) | Electric field strength (V/cm) | Pulse length (ms) | Calorie strength (J/100μl) total | Calorie strength (J/100μl) per one pulse | |
| 93 | 4mm | 0.10 | 200 | - | - | - | - | - | | - |
| 94 | 4mm | 0.10 | 200 | 65 | 125 | 312.5 | 5 | 0.601 | 0.601 | 50 |
| 95 | 4mm | 0.10 | 200 | 67 | 185 | 462.5 | 5 | 1.277 | 1.277 | 50 |
| 96 | 4mm | 0.10 | 200 | 65 | 250 | 625 | 5 | 2.404 | 2.404 | 50 |
| 97 | 4mm | 0.10 | 400 | 38 | 90 | 225 | 5 | 0.266 | 0.266 | 50 |
| 98 | 4mm | 0.10 | 400 | 36 | 125 | 312.5 | 5 | 0.543 | 0.543 | 50 |
| 99 | 4mm | 0.10 | 400 | 37 | 150 | 375 | 5 | 0.760 | 0.760 | 50 |
| 100 | 4mm | 0.10 | 800 | 23 | 60 | 150 | 5 | 0.098 | 0.098 | 50 |
| 101 | 4mm | 0.10 | 800 | 18 | 90 | 225 | 5 | 0.281 | 0.281 | 50 |
| 102 | 4mm | 0.10 | 800 | 19 | 125 | 312.5 | 5 | 0.514 | 0.514 | 50 |

[Table 22-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse internal (ms) | Number of pulses | Calorie strength (J/100 μl) total | Calorie strength (J/100 μl) per one pulse | | |
| 93 | - | - | - | - | - | - | - | >95 | 0 |
| 94 | 20 | 50 | 50 | 50 | 10 | 1.538 | 0.154 | 90 | 0 |
| 95 | 30 | 75 | 50 | 50 | 10 | 3.358 | 0.336 | >90 | 64 |
| 96 | 40 | 100 | 50 | 50 | 10 | 6.154 | 0.615 | 90 | 66 |
| 97 | 15 | 37.5 | 50 | 50 | 10 | 0.740 | 0.074 | >90 | 0 |
| 98 | 20 | 50 | 50 | 50 | 10 | 1.389 | 0.139 | >90 | 0 |
| 99 | 25 | 62.5 | 50 | 50 | 10 | 2111 | 0.211 | 90 | 10 |
| 100 | 10 | 25 | 50 | 50 | 10 | 0.272 | 0.027 | >90 | 0 |
| 101 | 15 | 37.5 | 50 | 50 | 10 | 0.781 | 0.078 | >90 | 0 |
| 102 | 20 | 50 | 50 | 50 | 10 | 1.316 | 0.132 | >90 | 0 |

EP 2 527 433 B1

76

<Example 23> Examination by using 4 mm gap cuvette (4)

As shown in Tables 23, electroporation was done by varying the buffer volume by using 4 mm gap cuvette. Other conditions for electroporation were same to Example 1 (samples 170-177).

Note that a sample, which was not subjected to electric pulse treatment after put into the cuvette, was used as a control (sample 169).

And the viability and the gene transferring rate were calculated in the same manner as in Example 1. The result is shown in Tables 23.

The result showed that the electroporating conditions of electric field strength and calorie strength at 100 $\mu$L in the case of using 2 mm gap cuvette were also applicable to the case where 4 mm gap cuvette was used and buffer volume was 100-800 $\mu$L.

[Table 23-A]

| Sample | Cuvette | DNA concentraction (μg/μl) | Volume (μl) | Electric impedance (Ω) | First electric pulse | | | | | Pulse internal (ms) |
|---|---|---|---|---|---|---|---|---|---|---|
| | | | | | Voltage (V) | Electric field strength (V/cm) | Pulse length (ms) | Calorie strength (J/100μl) total | Calorie strength (J/100μl) per one pulse | |
| 169 | 4mm | 0.10 | 200 | - | - | - | - | - | - | - |
| 170 | 4mm | 0.10 | 100 | 146 | 210 | 525 | 5 | 1.510 | 1.510 | 50 |
| 171 | 4mm | 0.10 | 200 | 75 | 210 | 525 | 5 | 1.470 | 1.470 | 50 |
| 172 | 4mm | 0.10 | 300 | 52 | 210 | 525 | 5 | 1.413 | 1.413 | 50 |
| 173 | 4mm | 0.10 | 400 | 37 | 210 | 525 | 5 | 1.490 | 1.490 | 50 |
| 174 | 4mm | 0.10 | 500 | 38 | 210 | 525 | 5 | 1.161 | 1.161 | 50 |
| 176 | 4mm | 0.10 | 000 | 28 | 210 | 525 | 5 | 1.313 | 1.313 | 50 |
| 176 | 4mm | 0.10 | 700 | 28 | 210 | 525 | 5 | 1.125 | 1.125 | 50 |
| 177 | 4mm | 0.10 | 800 | 10 | 210 | 525 | 5 | 1.451 | 1.451 | 50 |

[Table 23-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100μl) total | Calorie strength (J/100 μl) per one pulse | | |
| 169 | - | - | - | - | - | - | - | >90 | 0 |
| 170 | 40 | 100 | 50 | 50 | 10 | 5.479 | 0.548 | 80 | 76 |
| 171 | 40 | 100 | 50 | 50 | 10 | 5.333 | 0.533 | 90 | 72 |
| 172 | 40 | 100 | 50 | 50 | 10 | 5.128 | 0.513 | 90 | 63 |
| 173 | 40 | 100 | 50 | 50 | 10 | 5.405 | 0.541 | 90 | 69 |
| 174 | 40 | 100 | 50 | 50 | 10 | 4.211 | 0.421 | 90 | 70 |
| 175 | 40 | 100 | 50 | 50 | 10 | 4.762 | 0.476 | 90 | 71 |
| 176 | 40 | 100 | 50 | 50 | 10 | 4.082 | 0.408 | 90 | 66 |
| 177 | 40 | 100 | 50 | 50 | 10 | 5.263 | 0.526 | 90 | 65 |

<Example 24> Examination of DNA concentration

As shown in Tables 24, electric pulses were applied by varying DNA concentration. Other conditions for electroporation were same to Example 1 (samples 193-202).

Note that a sample, which was not subjected to electric pulse treatment after put into the cuvette, was used as a control (sample 192).

And the viability and the gene transferring rate were calculated in the same manner as in Example 1. The result is shown in Tables 24.

The result showed that the gene transferring rate was going up but the viability was going down as the DNA concentration was increasing, but that the viability was going up but the gene transferring rate was going down as the DNA concentration was decreasing.

Specifically it suggested that the DNA concentration more than 0.01 $\mu$g/$\mu$L, especially 0.03-0.5 $\mu$g/$\mu$L was suitable.

[Table 24-A]

| Sample | Cuvette | DNA concentration (μg/μl) | Volume (μl) | Electric impedance (Ω) | First electric pulse | | | | | |
| --- | --- | --- | --- | --- | --- | --- | --- | --- | --- | --- |
| | | | | | Voltage (V) | electric field strength (V/cm) | Pulse length (ms) | Calorie strength (J/100μl) total | Calorie strength (J/100 μl) per one pulse | Pulse interval (ms) |
| 192 | 2mm | 0.10 | 100 | - | - | - | - | - | - | - |
| 193 | 2mm | 0.01 | 100 | 47 | 125 | 625 | 5 | 1.662 | 1.662 | 50 |
| 194 | 2mm | 0.03 | 100 | 51 | 125 | 025 | 5 | 1.532 | 1.532 | 50 |
| 195 | 2mm | 0.05 | 100 | 49 | 125 | 625 | 5 | 1.594 | 1.594 | 50 |
| 196 | 2mm | 0.07 | 100 | 48 | 125 | 625 | 5 | 1.628 | 1.628 | 50 |
| 197 | 2mm | 0.10 | 100 | 46 | 125 | 625 | 5 | 1.698 | 1.698 | 50 |
| 198 | 2mm | 0.15 | 100 | 48 | 125 | 625 | 5 | 1.628 | 1.628 | 50 |
| 199 | 2mm | 0.20 | 100 | 58 | 125 | 625 | 5 | 1.347 | 1.347 | 50 |
| 200 | 2mm | 0.30 | 100 | 59 | 125 | 625 | 5 | 1.324 | 1.324 | 50 |
| 201 | 2mm | 0.40 | 100 | 78 | 125 | 625 | 5 | 1.002 | 1.002 | 50 |
| 202 | 2mm | 0.50 | 100 | 75 | 125 | 625 | 5 | 1.042 | 1.042 | 50 |

[Table 24-B]

| Sample | Second electric pulse | | | | | | | Viability (%) | Gene transferring rate (%) |
|---|---|---|---|---|---|---|---|---|---|
| | voltage (V) | Electric field strength (V/cm) | Pulse length (ms) | Pulse interval (ms) | Number of pulses | Calorie strength (J/100μl) total | Calorie strength (J/100μl) per one pulse | | |
| 192 | - | - | - | - | - | - | - | >95 | 0 |
| 193 | 20 | 100 | 50 | 50 | 10 | 4.255 | 0.426 | >95 | 39 |
| 194 | 20 | 100 | 50 | 50 | 10 | 3.922 | 0.392 | >95 | 72 |
| 195 | 20 | 100 | 50 | 50 | 10 | 4.082 | 0.408 | 90 | 74 |
| 196 | 20 | 100 | 50 | 50 | 10 | 4.167 | 0.417 | 90 | 82 |
| 197 | 20 | 100 | 50 | 50 | 10 | 4.348 | 0.435 | 90 | 92 |
| 198 | 20 | 100 | 50 | 50 | 10 | 4.167 | 0.417 | 80 | 93 |
| 199 | 20 | 100 | 50 | 50 | 10 | 3.448 | 0.345 | 80 | 90 |
| 200 | 20 | 100 | 50 | 50 | 10 | 3.390 | 0.339 | 70 | 91 |
| 201 | 20 | 100 | 50 | 50 | 10 | 2.564 | 0.256 | 70 | 93 |
| 202 | 20 | 100 | 50 | 50 | 10 | 2.667 | 0.267 | 70 | 93 |

<Example 25> Examination of serum concentration

As shown in Tables 25, TIG-7 cells (human embryonic lung cells) were used as the targeted cells, and antibiotic-free ES media were used as electroporation buffers by varying serum concentrations. Other conditions for electroporation were same to Example 1 (samples 204-207).

Note that a sample, which was not subjected to electric pulse treatment after put into the cuvette, was used as a control (sample 203).

And the viability and the gene transferring rate were calculated in the same manner as in Example 1. The result is shown in Tables 25.

The result showed that the gene transferring rate was decreased when serum was contained in the medium. This result suggested that it was preferred to use a serum-free medium obtained by removing serum in the case where a medium is used as an electroporation buffer.

This result also suggested that the same conditions as in the case of Hela cells (human cervical cancer cells) as cancer cells were applicable to TIG-7 cells (human embryonic lung cells) as normal cells.

[Table 25-A]

| Sample | Cuvette | DNA concentration (μg/μl) | Serum concentration (%) | Volume (μl) | electric impedance (Q) | First electric pulse | | | | | Pulse interval (ms) |
| | | | | | | Voltage (V) | Electric field strength (V/cm) | Pulse length (ms) | Calorie strength (J/100 μl) total | Calorie strength (J/100 μl) per one pulse | |
|---|---|---|---|---|---|---|---|---|---|---|---|
| 203 | 2mm | 0.10 | 0 | 100 | - | - | - | - | - | - | - |
| 204 | 2mm | 0.10 | 0 | 100 | 40 | 110 | 560 | 30 | 0.075 | 9.075 | 50 |
| 205 | 2mm | 0.10 | 1 | 100 | 50 | 110 | 550 | 30 | 7.260 | 7.260 | 50 |
| 206 | 2mm | 0.10 | 5 | 100 | 41 | 110 | 550 | 30 | 8.854 | 8.854 | 50 |
| 207 | 2mm | 0.10 | 10 | 100 | 45 | 110 | 550 | 30 | 8.067 | 8.067 | 50 |

[Table 25-B]

| Sample | Second Electric pulse | | | | | | | Viability (%) | Gene transferring rate (%) |
| | Voltage (V) | Electric field strength (V/cm) | Pulse length (ms) | Pulse interval (ms) | Number of pulses | Calorie strength (J/100μl) total | Calorie strength (J/100μl) per one pulse | | |
|---|---|---|---|---|---|---|---|---|---|
| 203 | - | - | - | - | - | - | - | 90 | 0 |
| 204 | 20 | 100 | 50 | 50 | 10 | 5.000 | 0.500 | 80 | 75 |
| 205 | 20 | 100 | *50* | 50 | 10 | 4.000 | 0.400 | 83 | 50 |
| 206 | 20 | 100 | 50 | 50 | 10 | 4.878 | 0.488 | 85 | 20 |
| 207 | 20 | 100 | 50 | 50 | 10 | 4.444 | 0.444 | 85 | 1.5 |

<Example 26> Examination to rat embryonic fibroblasts

As shown in Tables 26, REF cells (rat embryonic fibroblasts) were used as the targeted cells, an Opti-MEM medium not containing any serum/antibiotic (serum and antibiotic free buffer) was used as an electroporation buffer, and electric pulses were applied by varying pulse length and DNA concentration. Other conditions for electroporation were same to Example 1 (samples 215-219).

Note that a sample, which was not subjected to electric pulse treatment after put into the cuvette, was used as a control (sample 208).

And the viability and the gene transferring rate were calculated in the same manner as in Example 1 except culturing was done by using a DMEM medium. The result is shown in Tables 26.

The result showed that the same conditions as in the case of the human Hela cells (human cervical cancer cells) were applicable to the rat REF cells (rat embryonic fibroblasts).

And it showed also that slightly higher total calorie strength of the first electric pulse was better to the REF cells than the case of the Hela cells.

[Table 26-A]

| Sample | Cuvette | DNA concentration ($\mu$g/$\mu$l) | Volume ($\mu$l) | Electric inpedancc (Q) | First electric pulse | | | | | Pulse interval (ms) |
| | | | | | Voltage (V) | Electric field strength (V/cm) | Pulse length (ms) | Calorie strength (J/100$\mu$l) total | Calorie strength (J/100/$\mu$l) per one pulse | |
|---|---|---|---|---|---|---|---|---|---|---|
| 208 | 2mm | 0.10 | 100 | - | - | - | - | - | - | - |
| 215 | 2mm | 0.10 | 100 | 45 | 275 | 1375 | 0.5 | 0.822 | 0.822 | 50 |
| 216 | 2mm | 0.10 | 100 | 52 | 275 | 1375 | 2 | 2.909 | 2.909 | 50 |
| 217 | 2mm | 0.10 | 100 | 54 | 275 | 1375 | 5 | 7.002 | 7.002 | 50 |
| 218 | 2mm | 0.05 | 100 | 54 | 275 | 1375 | 5 | 7.002 | 7.002 | 50 |
| 219 | 2mm | 0.02 | 100 | 54 | 275 | 1375 | 5 | 7.002 | 7.002 | 50 |

[Table 26-B]

**Second electric pulse**

| Sample | Voltage (V) | Electric field strength (V/cm) | Pulse length (ms) | Pulse interval (ms) | Number of pulses | Calorie strength (J/100μl) total | Calorie strength (J/100μl) per one pulse | Viability (%) | Gene transferring rate (%) |
|---|---|---|---|---|---|---|---|---|---|
| 208 | - | - | - | - | - | - | - | 100 | 0 |
| 215 | 20 | 100 | 50 | 50 | 10 | 4.348 | 0.435 | 95 | 40 |
| 216 | 20 | 100 | 50 | 50 | 10 | 3.846 | 0.385 | 95 | 60 |
| 217 | 20 | 100 | 50 | 50 | 10 | 3.704 | 0.370 | 90 | 99 |
| 218 | 20 | 100 | 50 | 50 | 10 | 3.704 | 0.370 | 90 | 95 |
| 219 | 20 | 100 | 50 | 50 | 10 | 3.704 | 0.370 | 90 | 70 |

<Example 27> Examination to human hepatoma cells

As shown in Tables 27, HepG2 cells (human hepatoma cells) were used as the targeted cells, a DMEM medium not containing any serum/antibiotic (serum and antibiotic free buffer) was used as an electroporation buffer, and electric pulses were applied by varying voltage, pulse length and DNA concentration. Other conditions for electroporation were same to Example 1 (samples 221-228).

Note that a sample, which was not subjected to electric pulse treatment after put into the cuvette, was used as a control (sample 220).

And the viability and the gene transferring rate were calculated in the same manner as in Example 1 except culturing was done by using a DMEM medium. The result is shown in Tables 27.

The result showed that the same conditions as in the case of the Hela cells (human cervical cancer cells) as cervical cancer were applicable to the HepG2 cells (human hepatoma cells) as hapatoma.

And it showed also that slightly lower electric field strength and higher calorie strength (longer pulse length) of the first electric pulse were better to the HepG2 cells than the case of the Hela cells.

[Table 27-A]

| Sample | Cuvette | DNA concentration ($\mu$g/$\mu$l) | Volume ($\mu$l) | Electric inpedance ($\Omega$) | First electric pulse | | | | | Pulse interval (ms) |
|---|---|---|---|---|---|---|---|---|---|---|
| | | | | | Voltage (V) | Electric field strength (V/cm) | Pulse length (ms) | Calorie strength (J/100 $\mu$l) total | Calorie strength (J/100 $\mu$l) per one pulse | |
| 220 | 2mm | 0.10 | 100 | - | - | - | - | - | - | - |
| 221 | 2mm | 0.10 | 100 | 46 | 110 | 550 | 75 | 19.728 | 19.728 | 50 |
| 222 | 2mm | 0.10 | 100 | 41 | 110 | 550 | 99 | 29.217 | 29.217 | 50 |
| 223 | 2mm | 0.10 | 100 | 40 | 125 | 625 | 15 | 5.859 | 5.859 | 50 |
| 224 | 2mm | 0.10 | 100 | 46 | 125 | 625 | 30 | 10.190 | - 10.190 | 50 |
| 225 | 2mm | 0.10 | 100 | 41 | 150 | 750 | 2 | 1.098 | 1.088 | 50 |
| 226 | 2mm | 0.10 | 100 | 47 | 150 | 750 | 5 | 2.394 | 2.394 | 50 |
| 227 | 2mm | 0.05 | 100 | 47 | 150 | 750 | 10 | 4.737 | 4.787 | 50 |
| 228 | 2mm | 0.02 | 100 | 45 | 200 | 1000 | 2 | 1.778 | 1.778 | 50 |

[Table 27-B]

| Sample | Second electric pulse | | | | | | | Viability (%) | Gene transferring rate (%) |
|---|---|---|---|---|---|---|---|---|---|
| | Voltage (V) | Electric field strength (V/cm) | Pulse length (ms) | Pulse interval (ms) | Number of pulses | Calorie strength (J/100 $\mu$l) total | Calorie strength (J/100 $\mu$l) per one pulse | | |
| 220 | - | - | - | - | - | - | - | >95 | 0 |
| 221 | 20 | 100 | 50 | 50 | 10 | 4.348 | 0.435 | 85 | 69 |
| 222 | 20 | 100 | 50 | 50 | 10 | 4.878 | 0.488 | 80 | 76 |
| 223 | 20 | 100 | 50 | 50 | 10 | 5.000 | 0.500 | 55 | 69 |
| 224 | 20 | 100 | 50 | 50 | 10 | 4.343 | 0.435 | 45 | 82 |
| 225 | 20 | 100 | 50 | 50 | 10 | 4.878 | 0.488 | 85 | 23 |
| 226 | 20 | 100 | 50 | 50 | 10 | 4.255 | 0.426 | 75 | 36 |
| 227 | 20 | 100 | 50 | 50 | 10 | 4.255 | 0.426 | 70 | 76 |
| 228 | 20 | 100 | 50 | 50 | 10 | 4.444 | 0.444 | 75 | 32 |

<Example 28> Application to various animal cells

Various cells (cell lines and primary cells) shown in Tables 28-32 were used, various serum/antibiotic-free growth media were used as electroporation buffers, and electric pulses suitable for various cells were applied. Other conditions for electroporation were same to Example 1. Typical electric pulse conditions for various cells are shown in Tables 28-32.

And the viability and the gene transferring rate were calculated in the same manner as in Example 1 except culturing was done by using various growth media. The results are shown in Tables 33-36.

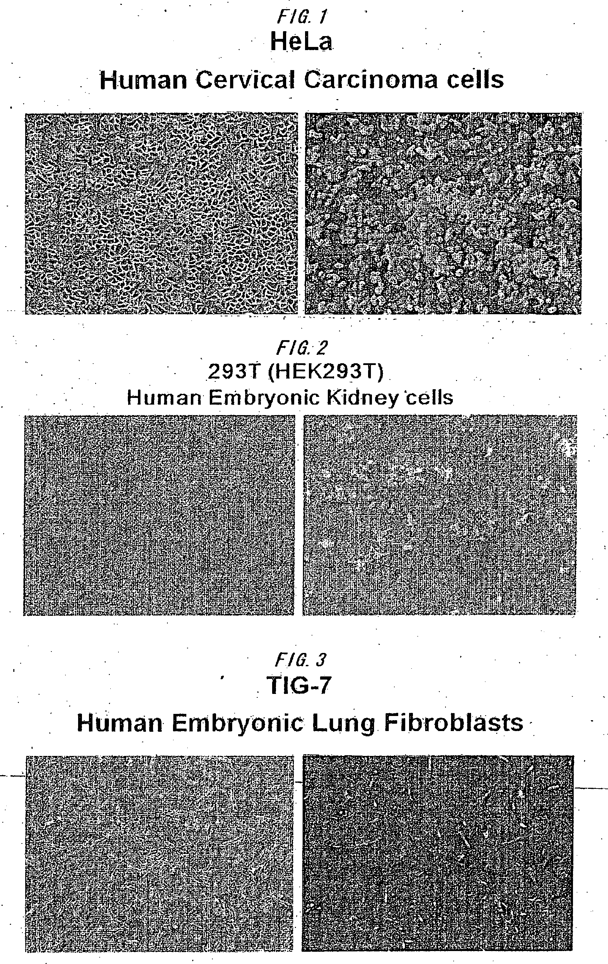

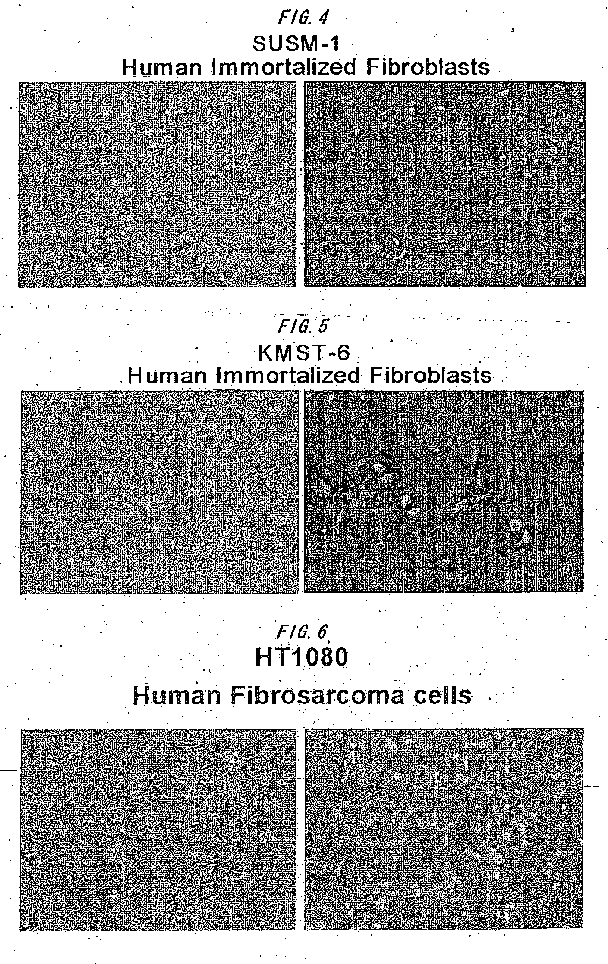

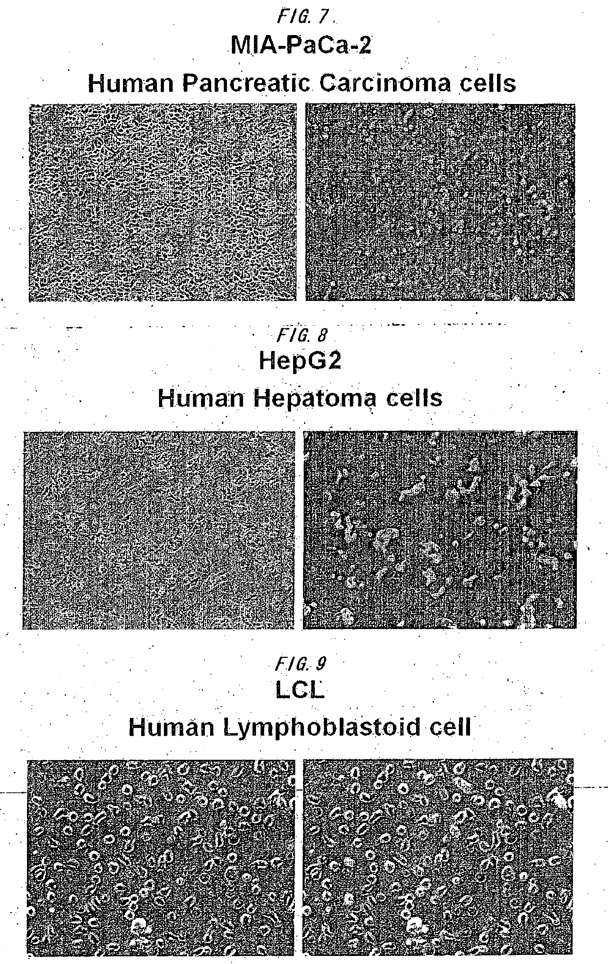

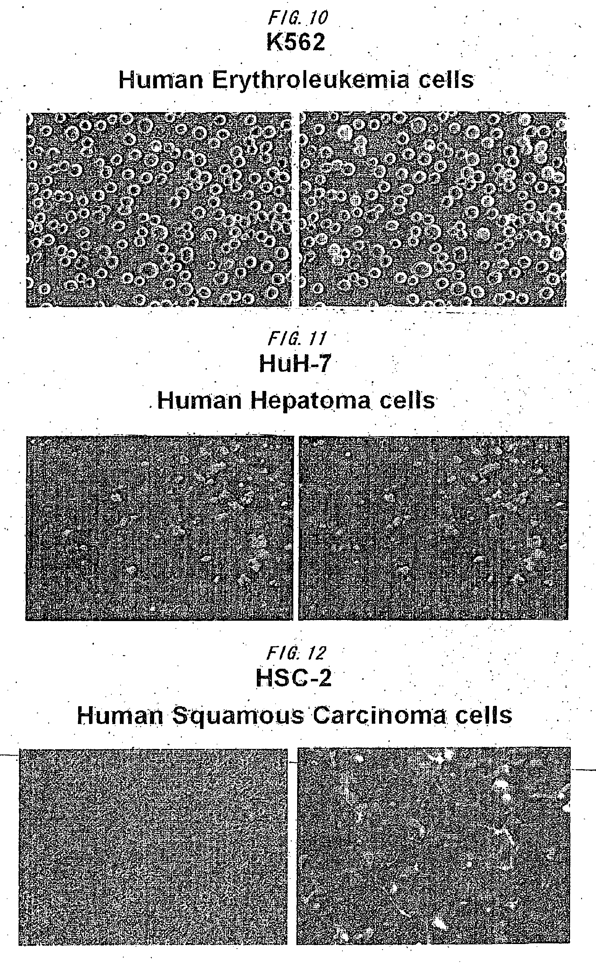

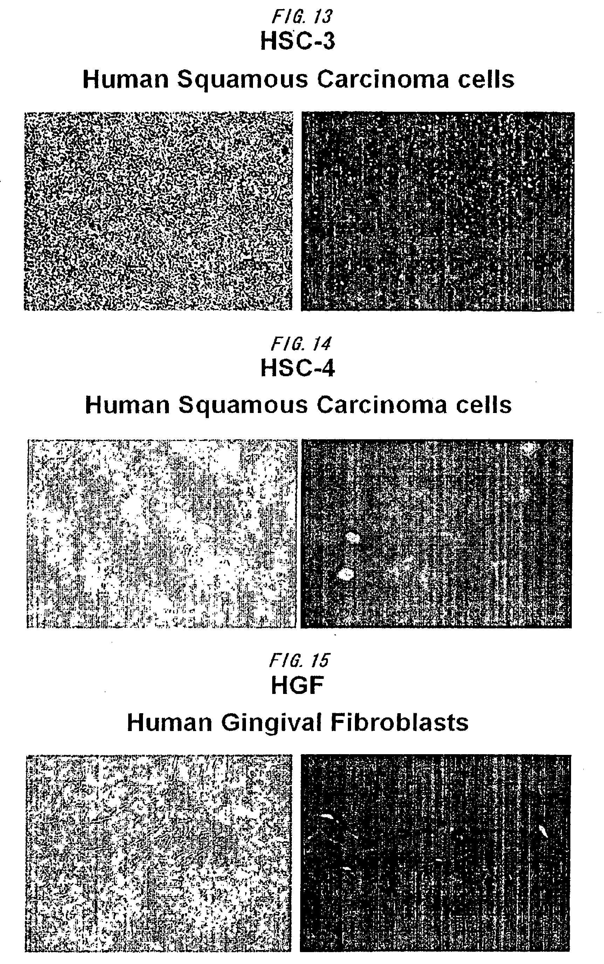

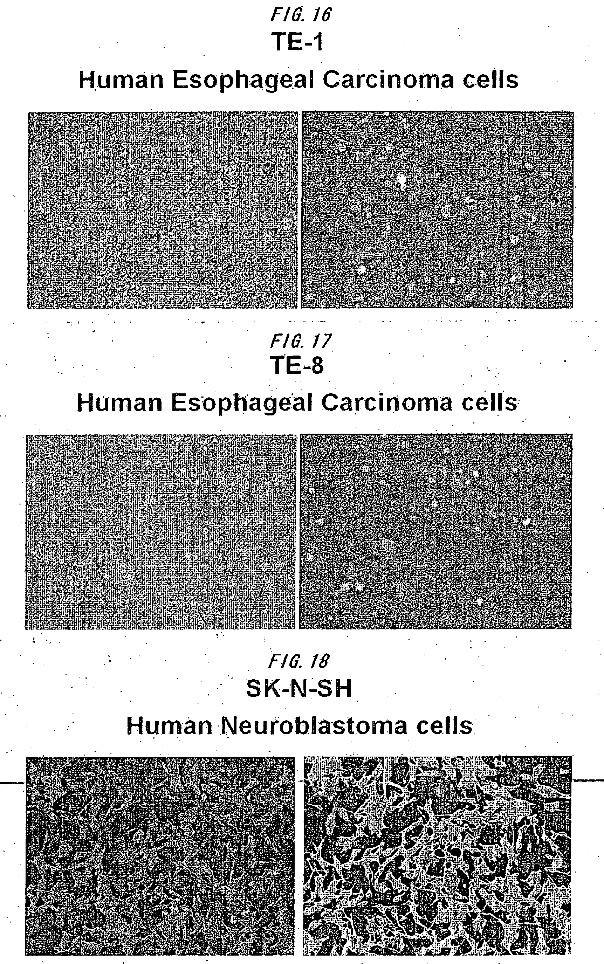

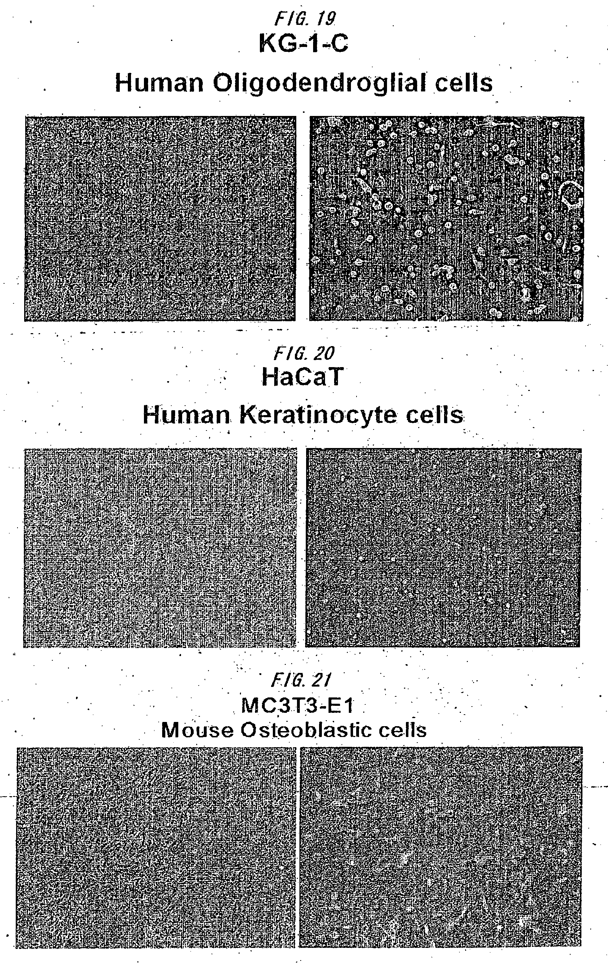

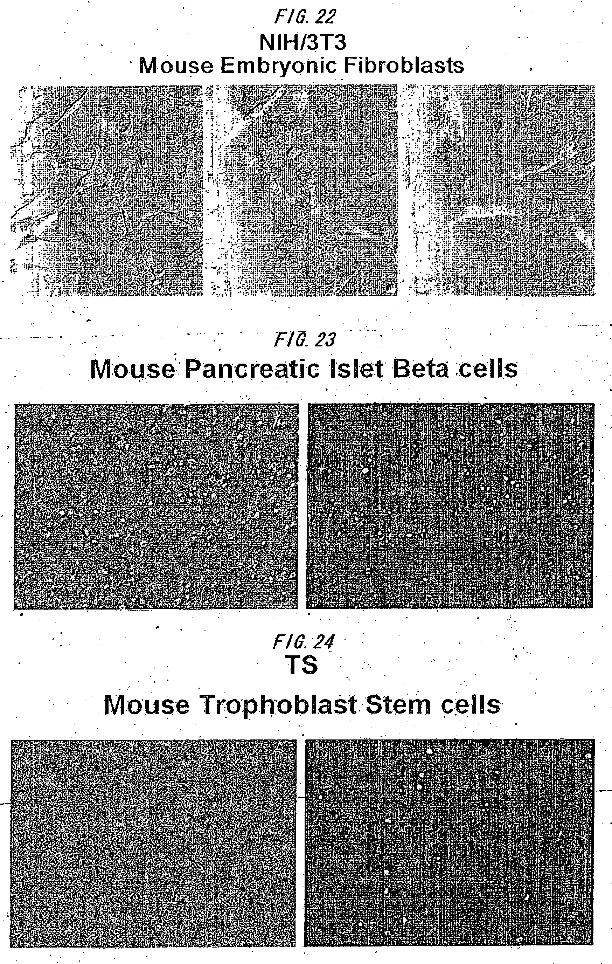

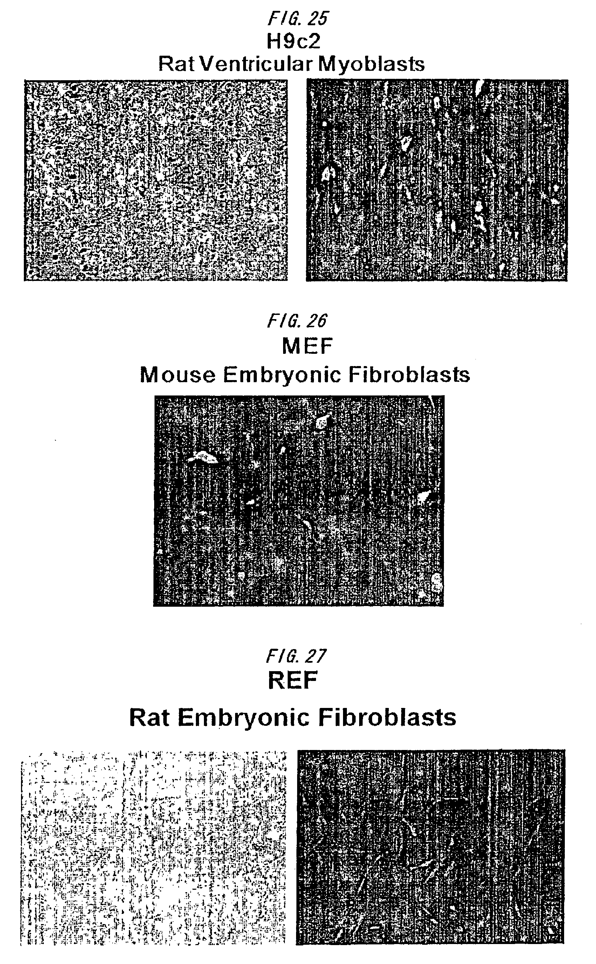

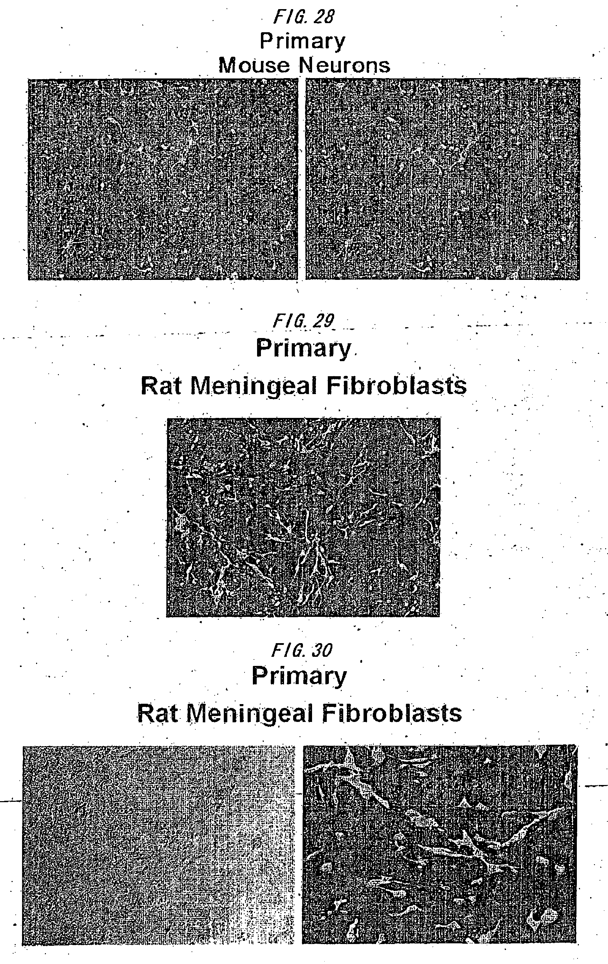

Further, the photo images of the cells are shown in FIGS. 1-35. In the photo images, the left side photo images each show the cells after culturing and the right side photo images each show the detected fluorescent-labeled protein of the transferred gene (Figs. 18, 22, 26, 28, 29 and 31 each show only the detected fluorescent-labeled protein).

[Table 28]

| <Hela/ Human Cervical Carcinoma cells: An example of human cancer cells> | | |
|---|---|---|
| Parameters | | Values |
| First electric pulse | Electric field strength | 625 V/cm |
| | Number of Pulses | 1 |
| | Calorie strength per one pulse | 2.170 J/100 μl |
| | Total calorie strength | 2.170 J/100 μl |
| Pulse interval | | 50 m sec |
| Second electric pulse | Electric field strength | 100 V/cm |
| | Number of Pulses | 10 |
| | Calorie strength per one pulse | 0.556 J/100 μl |
| | Total calorie strength | 5.556 J/100 μl |

[Table 29]

| <293T(HEK293T) Human Embryonic Kidney cells: An example of human normal cells> | | |
|---|---|---|
| Parameters | | Values |
| First electric pulse | Electric field strength | 625 V/cm |
| | Number of Pulses | 1 |
| | Calorie strength per one pulse | 4.006 J/100 μl |
| | Total calorie strength | 4.006 J/100 μl |
| Pulse interval | | 50 m sec |
| Second electric pulse | Electric field strength | 100 V/cm |
| | Number of Pulses | 10 |
| | Calorie strength per one pulse | 0.513 J/100 μl |
| | Total calorie strength | 5.128 J/100 μl |

[Table 30]

| <REFRat Embryonic Fibroblasts: An example of mouse/rat cells> | | |
|---|---|---|
| Parameters | | Values |
| First electric pulse | Electric field strength | 1375 V/cm |
| | Number of Pulses | 1 |
| | Calorie strength per one pulse | 7.002 J/100 μl |
| | Total calorie strength | 7.002 J/100 μl |
| Pulse interval | | 50 m sec |
| Second electric pulse | Electric field strength | 100 V/cm |
| | Number of Pulses | 10 |
| | Calorie strength per one pulse | 0.370 J/100 μl |
| | Total calorie strength | 3.704 J/100 μl |

[Table 31]

| <SACT-1 ddy Mouse ES cells (XY) : An example of ES cells> | | |
|---|---|---|
| Parameters | | Values |
| First electric pulse | Electric field strength | 625 V/cm |
| | Number of Pulses | 1 |
| | Calorie strength per one pulse | 2.232 J/100 μl |
| | Total calorie strength | 2.232 J/100 μl |
| Pulse interval | | 50 m sec |
| Second electric pulse | Electric field strength | 100 V/cm |
| | Number of Pulses | 10 |
| | Calorie strength per one pulse | 0.571 J/100 μl |
| | Total calorie strength | 5.714 J/100 μl |

[Table 32]

| <Mouse Neurons (Embryonic Day 14 Mouse Cerebral Cortex): An example of primary cells> | | |
|---|---|---|
| Parameters | | Values |
| First electric pulse | Electric field strength | 1375 V/cm |
| | Number of Pulses | 1 |
| | Calorie strength per one pulse | 2.140 J/100 μl |
| | Total calorie strength | 2.140 J/100 μl |
| Pulse interval | | 50 m sec |
| Second electric pulse | Electric field strength | 100 V/cm |
| | Number of Pulses | 10 |
| | Calorie strength per one pulse | 0.377 J/100 μl |
| | Total calorie strength | 3.773 J/100 μl |

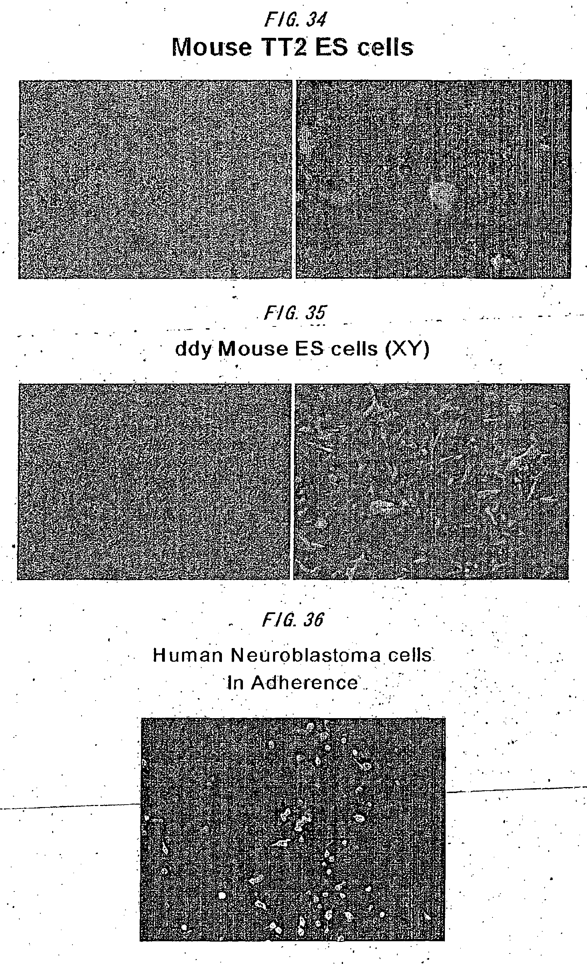

These results showed that the electroporation technique involving applying the first electric pulse and the second electric pulse under the above conditions was applicable to the cell lines and the primary cells originated from various tissues as well. For example, the results showed that electroporation was able to be performed with high viability and gene transferring rate in neural cells (Figs. 18, 19, 28 and 33) and ES cells (Figs. 34 and 35) as well.

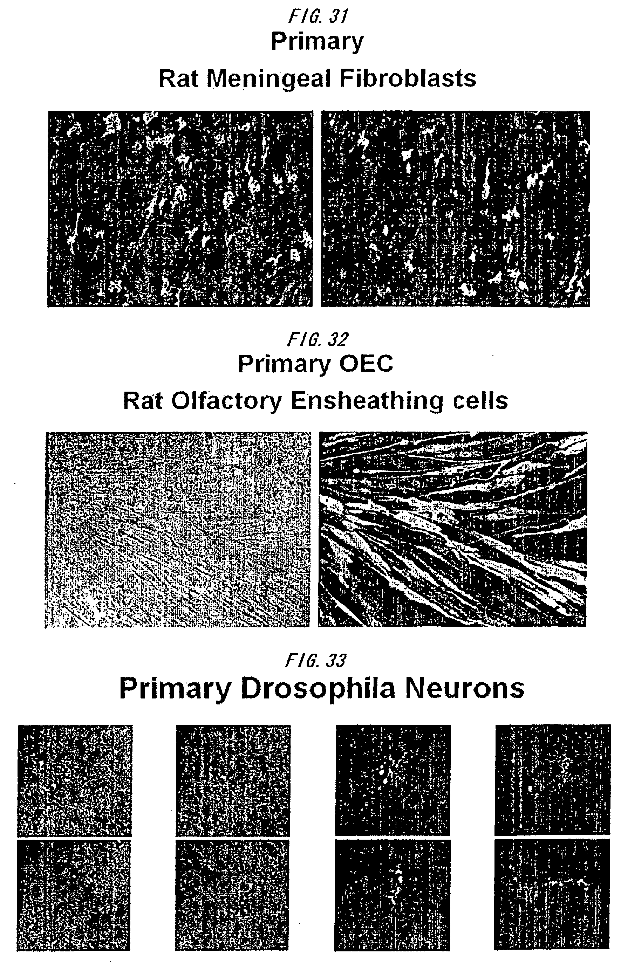

Further, the results show that the electroporation technique is applicable not only to mammals such as humans, mice, rats, dogs, and horses but also to drosophila as an insect (Fig. 33), suggesting the applicability to general animals.

[Table 33]

| Cell Name | Description/ Species | Characteristics, etc. | Viability | Efficiency | Photo Image |
|---|---|---|---|---|---|
| HeLa | Human Cervical Carcinoma cells | Epithelial, Immortalized, Adherent | 86% | 96% | Fig. 1 |
| 293T (HEK293T) | Human Embryonic Kidney cells | Epithelial, Adherent, SV40 large T antigen | 90% | 90% | Fig. 2 |
| TIG-7 | Human Embryonic Lung Fibroblasts | Normal Diploid Fibroblasts, Adherent | 89% | 76% | Fig. 3 |
| SUSM-1 | Human Immortalized Fibroblasts | Somewhat epithelial, Normal cells, Immortalized, Adherent | 77% | 71% | Fig. 4, |
| KMST-6 | Human Immortalized Fibroblasts | Somewhat epithelial, Normal cells, Immortalized, Adherent | 70% | 60% | Fig. 5 |
| HT1080 | Human Fibrosarcoma cells | Adherent, Invasive cancer cells | 93% | 81% | Fig. 6 |
| MIA-PaCa-2 | Human Pancreatic Carcinoma cells | Epithelial, Adherent | 80% | 77% | Fig. 7 |
| HepG2 | Human Hepatoma cells | Epithelial, Adherent, Well-differentiated liver cancer cells | 80% | 76% | Fig. 8 |
| HuH-7 | Human Hepatoma cells | Epithelial, Adherent, Well-differentiated liver cancer cells | 70% | 60% | Fig. 11 |
| H1299 (NCI-H1299) | Human Lung Cancer cells | Epithelial, Adherent, p53 qene defect | 90% | 90% | |
| HSC-2 | Human Squamous Carcinoma cells | Oral, Adherent | 90-95% | 98% | Fig. 12 |
| HSC-3 | Human Squamous Carcinoma cells | Tongue, Adherent | 90-95% | 98% | Fig. 13 |
| HSC-4 | Human Squamous Carcinoma cells | Tongue, Adherent | 80% | 34% | Fig. 14 |
| HGF | Human Gingival Fibroblasts | Normal cells, Adherent | Good | Good | Fig. 15 |
| MCF-7 | Human Breast Cancer cells | Epithelial, Polygonal, Adherent, Metastatic exudative pleural effusion breast cancer cells | 90% | 90-95% | |

(continued)

| Cell Name | Description/ Species | Characteristics, etc. | Viability | Efficiency | Photo Image |
|---|---|---|---|---|---|
| T47D | Human Breast Cancer cells | Epithelial, Adherent, Invasive ductal breast cancer cells | 90% | 80-90% | |
| A549 | Human Lung Adenocarcinoma cells | Epithelial, Adherent | 80-90% | 90% | |

[Table 34]

| Cell Name | Description, Species | Characteristics , etc. | Viability | Efficiency | Photo Image |
|---|---|---|---|---|---|
| TE-1 | Human Esophageal Carcinoma cells | Polyangular, Adherent, Esophagus cancer primary focus | 80-90% | 41% | Fig. 16 |
| TE-8 | Human Esophageal Carcinoma cells | Polyangular, Adherent, Esophagus cancer primary focus | 80-90% | 40% | Fig. 17 |
| LNCaP | Human Prostate Carcinoma cells | Epithelial, Adherent | 71% | 90.3% | |
| SK-N-SH | Human Neuroblastoma cells | Adherent | 95% | 95% | Fig. 18 |
| KG-1-C | Human Oligodendroglial cells | Adherent | 85% | 60% | Fig. 19 |
| HaCaT | Human Keratinocyte cells | Normal cells, Immortalized, Adherent | 40% | 80% | Fig. 20 |
| Hs52.sk | Human Skin Fibroblasts | Adherent | 38.5% | 10.8% | |
| iHAM-4 | Human Amniotic Mesenchymal cells | Adherent | 59% | 95% | |
| iHAE-7 | Human Amniotic Epithelial cells | Adherent | 70% | 40% | |
| Jurkat | Human T-cell Leukemia cells | Lymphoid, Suspension, IL-2 production | 81.8% | 38.3% | |
| Nalm-6 | Human B-cell Precursor Leukemia cells | Suspension | 65% | 63% | |
| Raj i | Human Burkitt's Lymphoma cells | B lymphocyte, Suspension, EB virus nuclear antigen positive | 74.1% | 52.6% | |
| LCL | Human Lymphoblastoid cells | Immortalized, Suspension | 41.2% | 40.4% | Fig. 9 |
| K562 | Human Erythroleukemia cells | Lymphoid, Suspension, NK-cell sensitivity | 34.4% | 42.4% | Fig. 10 |
| U937 | Human Histiocytic Lymphoma cells | Monocytoid, Suspension, Histiocytic lymphoma | 96% | 15% | |
| CMK-85 | Human Megakaryoblastic Leukemia cells | Suspension, Partially adherent, Down syndrome | 50.2% | 12.9% | |

[Table 35]

| Cell Name | Description, Species | Characteristics, etc. | Viability | Efficiency | Photo Image |
|---|---|---|---|---|---|
| NIH/3T3 | Mouse Embryonic Fibroblasts | Spindle-shaped, Fibroblastic, Immortalized, Adherent | Good | 54.7% | Fig. 22 |
| MEF | Mouse Embryonic Fibroblasts | Adherent | 95% | 60-70 % | Fig. 26 |
| MC3T3-E1 | Mouse Osteoblastic cells | Fibroblastic, Adherent, Skull | 40% | 80% | Fig. 21 |
| MS-1 | Mouse Pancreatic Endothelial cells | Adherent, Vascular endothelial | 90% | 90% | |
| | ddy Mouse Endometrial cells | Fibroblasts, Adherent | 60% | 80% | |
| | Mouse Pancreatic Islet Beta cells | Suspension | 60% | 40% | Fig. 23 |
| MEL | Mouse Erythroleukemia cells | Spleen origin, Spherical, Suspension | 70% | 50% | |
| BMMC | Mouse Bone Marrow-Derived Mast cells | Suspension | 35% | 26% | |
| mDC | Mouse Myeloid Dendritic cells | Suspension | 79.4% | 71.9% | |
| TS | Mouse Trophoblast Stem cells | Adherent | 59% | 47% | Fig. 24 |
| TT2 | Mouse TT2 ES cells | Adherent | 50-60 % | 50-60 % | Fig. 34 |
| SACT-1 | ddy Mouse ES cells (XY) | Adherent | 30% | 50% | Fig. 35 |
| PC12 | Rat Adrenal Pheochromocytoma cells | Epitherial, Weak adherent, Sympathetic neuron-like (NGF) | 70% | 50% | |
| H9c2 | Rat Ventricular Myoblasts | Skeletal muscle-like, Cardiac muscle-like, Adherent | 80% | 40% | Fig. 25 |
| REF | Rat Embryonic Fibroblasts | Adherent | 90% | 99% | Fig. 27 |
| | Rat WDA ES-like cells | Adherent | 60% | 80% | |
| CHO | Chinese Hamster Ovary cells | Fibroblasts, Epithelial, Adherent | 70% | 90% | |
| MDCK | Madrin-Darby Canine Kidney cells | Epithelial, Adherent | Good | 17.8% | |

[Table 36]

| Cell Name | Description, Species | Characteristics, etc. | Viability | Efficiency | Photo Image |
|---|---|---|---|---|---|
| | Human Amniotic Mesenchymal cells | Primary, Adherent | 70% | 40% | |
| | Mouse Neurons (Embryonic day 14 cerebral cortex) | Primary, Adherent | 80% | 50% | Fig. 28 |
| | Mouse Neurons (Embryonic day 16.5 hippocampus) | Primary, Adherent | 50% | 20% | |

(continued)

| Cell Name | Description, Species | Characteristics, etc. | Viability | Efficiency | Photo Image |
|---|---|---|---|---|---|
| | Rat Meningeal Fibroblasts (Postnatal day 3) | Primary, Adherent | 90% | 95% | Figs. 29, 30 & 31 |
| OEC | Rat Olfactory Ensheathing cells (Postnatal week 3) | Primary, Adherent | 93% | 46% | Fig. 32 |
| | Drosophila Neurons | Primary, Adherent | 50% | 31% | Fig. 33 |
| | Horse Monocyte-Derived Dendritic cells | Primary, Suspension | Good | Good | |

<Example 29> Application to adherent cells

First, SH-SY5Y cells (human neuroblastoma cells: adherent cells) cultured in a 12-well plate were washed twice with PBS. Next, 240-350 $\mu$L of an electroporation buffer containing 1 $\mu$g/$\mu$L of DNA (pCMV-EGFP vector) was put into the wells and an adherent cell electrode with legs (CUC513-5 electrode) was set on the cells.

The cells in an adherent state were then subjected to electroporation by carrying out electric pulse treatment under the conditions shown in Table 37.

The viability and the gene transferring rate were then calculated in the same manner as in Example 1 except culturing was done continuously after the liquid was exchanged to a DMEM medium. The result is shown in Table 38.

Further, a photo image of the cells is shown in Fig. 36. In the figure, the photo image shows the detected fluorescent-labeled protein.

[Table 37]

| <SH-SY5Y Human Neuroblastoma cells: Adherent state> | | |
|---|---|---|
| Parameters | | Values |
| First electric pulse | Electric field strength | 400 V/cm |
| | Number of Pulses | 1 |
| | Calorie strength per one pulse | 1.980 J/100 $\mu$l |
| | Total calorie strength | 1.980 J/100 $\mu$l |
| Pulse interval | | 50 m sec |
| Second electric pulse | Electric field strength | 60 V/cm |
| | Number of Pulses | 10 |
| | Calorie strength per one pulse | 0.093 J/100 $\mu$l |
| | Total calorie strength | 0.928 J/100 $\mu$l |

From these results it was shown that the electroporation technique involving applying the first electric pulse and the second pulse under the above conditions was directly applicable to the cells in an adherent state.

[Table 38]

| Cell Name | Description, Specie | Characteristics, etc. | Viability | Efficiency | Photo Image |
|---|---|---|---|---|---|
| SH-SY5Y | Human Neuroblastoma cells | Adherent | 90% | 50% | Fig. 36 |

Industrial Applicability

This invention is expected to be usefully applied to experiments and research in a wide range of industrial fields such as medical, food, agricultural and other fields.

Further, this invention allows useful animal gene transferred cells (e.g., iPS cells, living stem cells) to be prepared efficiently at low cost and to be applied to a wide range of industrial fields.

**Claims**

1. A method for transferring an extraneous gene into an animal cell by an electroporation technique, the method comprising:

   (a) preparing a solution containing suspended animal cells and the extraneous gene to be transferred into the animal cell;
   (b) filling a 1mm gap cuvette electrode, a 2mm gap cuvette electrode, or a 4mm gap cuvette electrode with the solution;
   (c) applying, to the animal cellsuspended in the solution, a first electric pulse having an electric field strength of 500 to 875 V/cm so that a total calorie strength is 1.39 to 4.37 J/100 $\mu$L; and
   (d) applying a second electric pulse having an electric field strength of 50 to 250 V/cm so that a calorie strength per pulse is 0.13 to 3.57 J/100 $\mu$L,

   wherein methods of treatment of the human or animal body by surgery or therapy are excluded.

2. A method for transferring an extraneous gene according to claim 1, wherein the applying of the second electric pulse is carried out two or more times.

3. A method for transferring an extraneous gene according to claim 1 or 2, wherein the applying of the second electric pulse is carried out less than one minute after the applying of the first electric pulse.

4. A method for transferring an extraneous gene according to any one of claims 1 to 3, wherein the animal cell is a mammalian cell.

**Patentansprüche**

1. Verfahren zur Transferierung eines Fremdgens in eine Tierzelle mittels einer Elektroporationstechnik, wobei das Verfahren umfasst:

   (a) Herstellung einer Lösung, beinhaltend suspendierte Tierzellen und das Fremdgen, das in die Tierzelle transferiert werden soll;
   (b) Befüllen einer 1 mm Spalt-Küvette-Elektrode, einer 2mm Spalt-Küvette-Elektrode oder einer 4mm Spalt-Küvette-Elektrode mit der Lösung;
   (c) Anlegen eines ersten elektrischen Pulses, der eine elektrische Feldstärke von 500 bis 875 V/cm hat an die in der Lösung suspendierten Tierzelle, so dass eine Gesamtkaloriestärke 1,39 bis 4,37 J/100 $\mu$L beträgt; und
   (d) Anlegen eines zweiten elektrischen Pulses, der eine elektrische Feldstärke von 50 bis 250 V/cm hat, so dass eine Gesamtkaloriestärke pro Puls 0,13 bis 3,57 J/100 $\mu$L beträgt,

   wobei Verfahren zur chirurgischen oder therapeutischen Behandlung des menschlichen oder tierischen Körpers ausgeschlossen sind.

2. Verfahren zur Transferierung eines Fremdgens nach Anspruch 1, wobei das Anlegen des zweiten elektrischen Pulses zweimal oder mehrmals durchgeführt wird.

3. Verfahren zur Transferierung eines Fremdgens nach Anspruch 1 oder 2, wobei das Anlegen des zweiten elektrischen Pulses weniger als eine Minute nach dem Anlegen des ersten elektrischen Pulses durchgeführt wird.

4. Verfahren zur Transferierung eines Fremdgens nach einem der Ansprüche 1 bis 3, wobei die Tierzelle eine Säugerzelle ist.

**Revendications**

1. Procédé de transfert d'un gène étranger dans une cellule animale par une technique d'électroporation, le procédé comprenant :

   (a) la préparation d'une solution contenant des cellules animales en suspension et le gène étranger devant être transféré dans la cellule animale ;
   (b) le remplissage d'une cuvette à électrodes espacées de 1 mm, d'une cuvette à électrodes espacées de 2 mm, ou d'une cuvette à électrodes espacées de 4 mm avec la solution ;
   (c) l'application à la cellule animale en suspension dans la solution d'une première impulsion électrique ayant une intensité de champ électrique de 500 à 875 V/cm, de sorte qu'un pouvoir calorifique total est de 1,39 à 4,37 J/100 $\mu$L ; et
   (d) l'application d'une seconde impulsion électrique ayant une intensité de champ électrique de 50 à 250 V/cm de sorte qu'un pouvoir calorifique par impulsion est de 0,13 à 3,57 J/100 $\mu$L,

   dans lequel les procédés de traitement du corps humain ou animal par chirurgie ou thérapie sont exclus.

2. Procédé de transfert d'un gène étranger selon la revendication 1, dans lequel l'application de la seconde impulsion électrique est effectuée deux fois ou plus.

3. Procédé de transfert d'un gène étranger selon la revendication 1 ou la revendication 2, dans lequel l'application de la seconde impulsion électrique est effectuée moins d'une minute après l'application de la première impulsion électrique.

4. Procédé de transfert d'un gène étranger selon l'une quelconque des revendications 1 à 3, dans lequel la cellule animale est une cellule de mammifère.

*FIG. 1*

## HeLa

## Human Cervical Carcinoma cells

*FIG. 2*

## 293T (HEK293T)

## Human Embryonic Kidney cells

*FIG. 3*

## TIG-7

## Human Embryonic Lung Fibroblasts

*FIG. 4*

SUSM-1
Human Immortalized Fibroblasts

*FIG. 5*

KMST-6
Human Immortalized Fibroblasts

*FIG. 6*

HT1080

Human Fibrosarcoma cells

*FIG. 7*

# MIA-PaCa-2
## Human Pancreatic Carcinoma cells

*FIG. 8*

# HepG2
## Human Hepatoma cells

*FIG. 9*

# LCL
## Human Lymphoblastoid cell

*FIG. 10*

## K562

## Human Erythroleukemia cells

*FIG. 11*

## HuH-7

## Human Hepatoma cells

*FIG. 12*

## HSC-2

## Human Squamous Carcinoma cells

*FIG. 13*

## HSC-3

## Human Squamous Carcinoma cells

*FIG. 14*

## HSC-4

## Human Squamous Carcinoma cells

*FIG. 15*

## HGF

## Human Gingival Fibroblasts

*FIG. 16*

## TE-1

## Human Esophageal Carcinoma cells

*FIG. 17*

## TE-8

## Human Esophageal Carcinoma cells

*FIG. 18*

## SK-N-SH

## Human Neuroblastoma cells

*FIG. 19*
# KG-1-C
## Human Oligodendroglial cells

*FIG. 20*
# HaCaT
## Human Keratinocyte cells

*FIG. 21*
# MC3T3-E1
## Mouse Osteoblastic cells

*FIG. 22*

# NIH/3T3
## Mouse Embryonic Fibroblasts

*FIG. 23*

## Mouse Pancreatic Islet Beta cells

*FIG. 24*

# TS
## Mouse Trophoblast Stem cells

*FIG. 25*
# H9c2
## Rat Ventricular Myoblasts

*FIG. 26*
# MEF
## Mouse Embryonic Fibroblasts

*FIG. 27*
# REF
## Rat Embryonic Fibroblasts

*FIG. 28*

Primary
Mouse Neurons

*FIG. 29*

Primary
Rat Meningeal Fibroblasts

*FIG. 30*

Primary
Rat Meningeal Fibroblasts

*FIG. 31*

## Primary
## Rat Meningeal Fibroblasts

*FIG. 32*

## Primary OEC
## Rat Olfactory Ensheathing cells

*FIG. 33*

## Primary Drosophila Neurons

*FIG. 34*

# Mouse TT2 ES cells

*FIG. 35*

## ddy Mouse ES cells (XY)

*FIG. 36*

## Human Neuroblastoma cells
## In Adherence

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Short Technical Report. *Biotechniques,* 1994, vol. 17 (6

- **KANDUSER, M. et al.** Mechanisms involved in gene electrotransfer using high- and low-voltage pulses -an in vitro study. *BIOELECTROCHEMISTRY,* 2009, vol. 74, 265-271